(19) Europäisches Patentamt
European Patent Office
Office européen des brevets

(11) **EP 3 222 341 A1**

(12) **EUROPÄISCHE PATENTANMELDUNG**

(43) Veröffentlichungstag:
**27.09.2017 Patentblatt 2017/39**

(21) Anmeldenummer: **17156674.8**

(22) Anmeldetag: **17.02.2017**

(51) Int Cl.:
**B01D 61/28** (2006.01)    **B01D 67/00** (2006.01)
**B01D 69/02** (2006.01)    **B01D 71/68** (2006.01)
**B01D 69/12** (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(30) Priorität: **17.02.2016 DE 102016102782**

(71) Anmelder: **B. Braun Avitum AG**
**34212 Melsungen (DE)**

(72) Erfinder:
• FRIEBE, Alexander
  **01705 Freital (DE)**

• **NAPIERALA, Roland**
  **33824 Werther (DE)**
• **BAIER-GOSCHÜTZ, Angela**
  **01819 Bad-Gottleuba - Berggießhübel (DE)**
• **GÄBLER, Juliane**
  **01896 Pulsnitz (DE)**
• **ULBRICHT, Matthias**
  **45143 Essen (DE)**
• **EMIN, Clélia Jade Eleónore Victoria**
  **44789 Bochum (DE)**

(74) Vertreter: **Winter, Brandl, Fürniss, Hübner, Röss, Kaiser, Polte - Partnerschaft mbB Patent- und Rechtsanwaltskanzlei Alois-Steinecker-Strasse 22 85354 Freising (DE)**

(54) **DIALYSEMEMBRAN UND VERFAHREN ZU IHRER HERSTELLUNG**

(57) Die vorliegende Erfindung betrifft eine Membran zur Reinigung von Blut bzw. Dialysemembran in Hohlfasermembran- oder Flachmembran-Geometrie, aus einem Komposit, welcher aus wenigstens einer Basismembran auf Basis wenigstens eines Polysulfons oder eines Polyphenylsulfons mit wenigstens einem porenbildenden hydrophilen Additiv und wenigstens einer auf der Basismembran angeordneten Funktionalschicht aufgebaut ist, wobei die Funktionalschicht aus wenigstens einem polymeren polykationischen Haftvermittler und wenigstens einem polymeren Polyanion gebildet ist, wobei die Basismembran aus einem Material gebildet ist, welches ausgewählt ist aus: einem Polysulfon [PSU], einem sulfonierten Polysulfon [SPSU], einem Polyethersulfon [PES], einem sulfonierten Polyethersulfon [SPES], einem Polyphenylsulfon [PPSU], einem sulfonierten Polyphenylsulfon [SPPSU]; sowie Mischungen daraus. Die erfindungsgemäßen Membranen weisen eine hohe Wasser-Permeabilität und niedrige BSA-Siebkoeffizienten auf.

Fig. 1

EP 3 222 341 A1

## Beschreibung

Die vorliegende Erfindung betrifft eine Dialysemembran gemäß dem Oberbegriff des Anspruchs 1 sowie gemäß Anspruch 14, ein Verfahren zur Herstellung derselben gemäß Anspruch 9 sowie einem Dialysemembran-Modul gemäß Anspruch 15.

Im Rahmen der vorliegenden Erfindung werden folgende Polymer-Abkürzungen verwendet:

| Abkürzung | Polymer |
|-----------|---------|
| DEXS | Dextransulfat |
| PA | Polyamid |
| PAA | Polyacrylsäure |
| PAN | Polyacrylnitril |
| PC | Polycarbonat |
| PEG* | Polyethylenglykol |
| PEI | Polyethylenimin |
| PEO* | Polyethylenoxid |
| PES | Polyethersulfon |
| PMMA | Polymethylmethacrylat |
| PPSU | Polyphenylsulfon |
| PUR | Polyurethan |
| PSU | Polysulfon |
| PVP | Poly(vinylpyrrolidon) |
| SPES | sulfoniertes Polyethersulfon |
| SPSU | sulfoniertes Polysulfon |
| SPPSU | sulfoniertes Polyphenylsulfon |
| * Bei PEG und PEO handelt es sich grundsätzlich um dasselbe Molekül. Für die Zwecke der vorliegenden Erfindung wird unter PEG ein Polymer verstanden, welches eine Molmasse unter 100 kDa aufweist. Ein Polymer mit einer Molmasse ab 100 kDa wird PEO genannt. | |

Die Dialyse zur Behandlung eines Patienten nach Nierenversagen wurde in den 1940er Jahren erstmals erfolgreich durchgeführt. Seit dieser Zeit haben sich die Dialysemembranen schnell weiterentwickelt. Insbesondere gelang eine Miniaturisierung von der Trommelniere, über den Plattendialysator bis zum heutigen modernen Hohlfaser-Dialysator.

Membranen für Hohlfaser-Dialysatoren werden heute im industriellen Maßstab in großer Anzahl mit reproduzierbar hoher Qualität hergestellt und in der Regel als Einmalsysteme in Form von Dialysemembran-Modulen angeboten. Dabei haben insbesondere nanokontrollierte Spinntechnologien zu einer wesentlichen Verbesserung geführt. Mittlerweile bilden Dialysemembranen aus Kunststoffen den Standard für eine kostengünstige Therapie. Weiterentwicklungen der Dialysemembranen haben zum Ziel, der natürlichen Funktion der glomerulären Membran der menschlichen Niere immer näher zu kommen, um eine optimale Behandlung bei Niereninsuffizienz, vollständigem Nierenversagen oder dem operativ oder traumatisch bedingten Fehlen der Nieren zu gewährleisten.

Bei der Weiterentwicklung von zur Dialyse geeigneten Membranen stehen eine hohe Biokompatibilität und eine hohe bzw. einstellbare Wasser-Permeabilität bei gleichzeitiger Realisierung der therapeutisch gewünschten Trennwirkung im Vordergrund. Aus klinischer Sicht ist eine Dialysebehandlung weiterhin von wachsender Bedeutung, da die Anzahl der zu behandelnden Patienten weltweit jährlich ansteigt, was einerseits durch die immer älter werdende Bevölkerung bedingt ist und andererseits wegen der ökonomischen Möglichkeiten und der Verfügbarkeit der Behandlung von Niereninsuffizienz auch in Entwicklungsländern in Zukunft ansteigen wird.

Darüber hinaus wird ein sogenanntes "Downscaling" von Dialysatoren angestrebt, was die vollständige Miniaturisierung der Dialysemembranen bis hin zu einem wenig Energie verbrauchenden Nierenimplantat als Fernziel bedeuten würde [vgl. Stefan Heinrich, H. Oliver Pfirrmann, Nanotechnologie für die Gesundheit, Gesundheitsvorsorge im Wandel, 2, 12 (2010)].

Im Stand der Technik ist bislang eine Vielzahl von Membranen, teilweise zur Ultrafiltration und teilweise zur Hämodialyse, beschrieben.

So beschreibt die US 2013/0 277 878 A1 ein Verfahren zur Herstellung einer Hohlfaser-Dialysemembran mittels eines Spinnverfahrens mit Phaseninversion.

Derartige Membranen sind aufgebaut aus wenigstens einem hydrophilen und wenigstens einem hydrophoben Polymer. Als geeignete hydrophile Polymere sind in der US 2013/0 277 878 A1 Polyvinylpyrrolidon (PVP), Polyethylenglycol, Polyglykolmonoester, wasserlösliche Zellulosederivate, Polysorbat und Polyethylenoxid-Polypropylenoxid-Copolymere offenbart.

Die hydrophoben Polymere können gemäß US 2013/0 277 878 A1 die folgenden sein: Polyamide (PA), Polyacrylsäuren (PAA), Polyarylethersulfone (PAES), Polyethersulfone (PES), Polysulfone (PSU), Polyarylsulfon (PASU), Polycarbonate (PC), Polyurethane (PUR).

Dokument DE 10 2004 008 220 B4 offenbart hydrophile Hohlfasermembranen für die Hämodialyse. Als Material ist genannt ein erstes synthetisches Polymer, welches ein hydrophobes Polymer, unter anderem PSU und/oder PES sein kann. Die Kompositmembranen der DE 10 2004 008 220 B4 enthalten ein hydrophiles zweites Polymer, welches ausgewählt werden kann aus der Gruppe PVP, PEG, Polyvinylalkohol, Polyglykolmonoester, Polysorbat, Carboxymethylzellulose oder einer Mischung oder einem Copolymer dieser Polymere.

Ferner sind für die sogenannten High-Flux-Dialysemembranen der DE 10 2004 008 220 B4 eine Ultrafiltrationsrate in Albuminlösung im Bereich von 25 bis 60 ml/(h x $m^2$ x mm Hg) sowie ein Siebkoeffizient für Cytochrom C von mindestens 0,8 in Kombination mit einem Siebkoeffizienten für Albumin von höchstens 0,005 offenbart, wobei die Hohlfasermembranen der DE 10 2004 008 220 B4 frei von Glyzerin und anderen Zusätzen ist, die die Poren in der Membranwand stabilisieren.

Ferner offenbart die US 6 042 783 A eine Hohlfasermembran aus einem PSU-Harz, bei welchem das das nicht näher beschriebene Permeationsverhältnis für die gesamten Proteine $\leq 1\ \%$ beträgt.

Ferner offenbart Dokument EP 1 634 611 B1 einen Blutreiniger mit einer Hohlfasermembran aus einem Polymer vom Polysulfontyp und PVP, welche unter anderem dadurch gekennzeichnet ist, dass höchstens 10 ppm PVP aus einem Gramm Hohlfasermembranen wässrig eluierbar sind. Als Polymere vom Polysulfontyp sind Polysulfon und Polyethersulfon beschrieben.

Ferner offenbart CN 201 669 064 U eine Basismembran aus einem Polysulfon mit einem nicht weiter charakterisierten "Polyose"-Film für medizinische Zwecke.

Ferner offenbart beispielsweise die WO 2013/156 598 A1 Ultrafiltrationsmembranen, welche wenigstens eine Substratschicht auf Basis von teilweise sulfoniertem Polyethersulfon als Polymer enthalten. Die in diesem Stand der Technik eingesetzten Polysulfone sind ausschließlich Polyethersulfone, welche teilweise sulfoniert sein können.

Ferner offenbart die WO 2013/156 597 A1 Nanofiltrationsmembranen aus einem Komposit aus einer Substratschicht, wenigstens einem teilweise sulfoniertem Polyethersulfon und wenigstens einer Filmschicht aus einem kationischen Polymer, wobei als kationische Polymere solche offenbart sind, welche Trimethylammoniumsalze als Seitengruppen enthalten.

Neben der oben aufgelisteten Patentliteratur gibt es noch eine Reihe von wissenschaftlicher Literatur, welche sich mit Membranen und insbesondere mit für die Hämodialyse geeigneten Membranen beschäftigt.

So beschreibt beispielsweise Blanco et al. (2002): J Appl Polymer Sci 84, 2461 die breite Verwendung von Polysulfonen zur Herstellung asymmetrischer Membranen für die Ultra- und Nanofiltration. Konkret wird der Einsatz von Polysulfon als Träger und Polyamid als Funktionalschicht für asymmetrische Membranen beschrieben. Blanco et al. zeigen ferner, dass durch Sulfonierung die Hydrophilie der an sich hydrophoben PSU-Polymere gesteigert werden kann.

Ferner beschreiben Li et al. (2008): J Membrane Sci 309, 45 zweischichtige Hohlfasermembranen zur Proteintrennung am Beispiel BSA/Hb. Als poröse innere Trägerschicht dient Polyethersulfon und die äußere Schicht besteht aus Sulfon, wobei die Polyethersulfon-Schicht stabil genug war, um die sulfonierte Polyethersulfon-Schicht zu tragen, so dass die Membranen erfolgreich zur Proteintrennung eingesetzt werden konnten.

Mahlicli et al. (2013): J Mater Sci: Med 24, 533 offenbaren eine Oberflächenmodifikation von auf Polysulfonen basierenden Dialysemembranen mit Layer-by-Layer (LbL)-Selbstaufbau von Polyethylenimin/Alginat-Heparin-Schichten. Gemäß Mahlicli et al. wird zunächst ein Polysulfon sulfoniert und dann ein Blend aus Polysulfon und sulfoniertem Polysulfon zu einer flachen Trägermembran auf einer Glasplatte ausgegossen, wobei als Lösungsmittel N-Methyl-2-pyrrolidon angegeben wird.

Der LbL-Aufbau erfolgt gemäß Mahlicli et al. auf der durch die eingeführten $SO_3^-$-Gruppen negativ geladenen PSU/SPSU-Membran. Hierzu wurde die Membran in eine Lösung von Polyethylenimin [PEI] getaucht und 10 Minuten inkubiert. Nach Einstellung des pH-Wertes auf einen hinreichenden Wert, um die protonierte Form des PEI zu erzeugen, wird überschüssiges PEI ausgewaschen und die Membran in eine Alginat-Lösung eingetaucht und 10 Minuten inkubiert. Überschüssiges Alginat wurde durch Waschen entfernt. Die abwechselnde PEI/Alginat-Tauchung wird mehrfach wiederholt, um einen Multilayer-Aufbau zu erreichen. Als letzte Schicht wurde die so mit PEI-Alginat-Komplexen beschichtete Trägermembran dann entweder in eine reine Heparin-Lösung oder in Alginat/Heparin-Lösung eingetaucht, um als letzte Schicht eine reine Heparin- oder eine Mischschicht aus Alginat und Heparin zu erhalten. Gemäß Mahlicli et al. waren die Permeationseigenschaften der von den Autoren hergestellten Membranen für Harnstoff, Vitamin $B_{12}$ und Lysozym vergleichbar mit industriellen AN69-Dialysemembranen. Die Plasmaproteinadsorption der Membranen war signifikant vermindert. Ebenso war die Thrombozytenaktivierung als Folge der antikoagulativen Beschichtung deutlich verringert.

Darüber hinaus beschreiben Malaisamy et al. (2005): Langmuir 21, 10587 Nanofiltrationsmembranen mit Polyethersulfon als Träger und einer LbL-Beschichtung mit Polystyrolsulfonat [PSS] und protoniertem Poly(allylamin) [PAH und/oder Poly(diallydimethylammoniumchlorid) [PDADMAC].

Kopec et al. (2011): J Membrane Sci 369, 59 beschreiben das Designen von Membranoberflächenladungen mittels LbL-Technik am Beispiel von Trägermembranen aus Polyimiden und Beschichtungen aus sulfoniertem Poly(etherketon) [SPEEK] sowie Supports aus PSU mit SPEEK-Beschichtung. Die Membranen sind als Hohlfasermembranen für die Ultrafiltration ausgebildet.

Schlussendlich beschreiben Kochan et al. (2010): Desalination 250, 1008 Hohlfasermembranen aus Polyethersulfonen, deren Oberfläche im LbL-Verfahren mit einem Polyelektrolyten, insbesondere Polyethylenimin und Polystyrolsulfonat, modifiziert wird. Als PEI kommt ein kommerzielles PEI mit einer Molmasse von 57 KDa zum Einsatz. Die Membranen dieses Standes der Technik können zur Abwasserreinigung verwendet werden. Beim Testen des Molekulargewichts-Cut-Off-Wertes mit Dextran hat sich gemäß Kochan et al. herausgestellt, dass bei den beschichteten Membranen eine starke Erhöhung des Rückhalts für Dextran bei gleichzeitigem Absinken der Permeabilität beobachtet wurde.

Ausgehend vom nächstkommenden Stand der Technik der Mahlicli et al. (2013) ist es die Aufgabe der vorliegenden Erfindung, eine Dialysemembran mit verbesserten Eigenschaften und hoher Biokompatibilität zur Verfügung zu stellen.

Die Lösung dieser Aufgabe erfolgt durch eine Dialysemembran gemäß Anspruch 1 und Anspruch 14. Verfahrenstechnisch wird die obige Aufgabe durch die Merkmale des Anspruchs 9 gelöst.

Ein Dialysemembran-Modul gemäß Anspruch 15 löst die Aufgabe ebenfalls.

Insbesondere betrifft die vorliegende Erfindung beispielsweise eine Dialysemembran in Hohlfasermembran- oder Flachmembran-Geometrie, aus einem Komposit, welcher aus wenigstens einer Basismembran auf Basis wenigstens eines Polysulfons mit wenigstens einem porenbildenden hydrophilen Additiv und wenigstens einer auf der Basismembran angeordneten Funktionalschicht aufgebaut ist, wobei die Funktionalschicht aus wenigstens einem polymeren polykationischen Haftvermittler und wenigstens einem polymeren Polyanion gebildet ist, dadurch gekennzeichnet, dass die Basismembran aus einem Material gebildet ist, welches ausgewählt ist aus: einem Polysulfon [PSU], einem sulfonierten Polysulfon [SPSU], einem Polyethersulfon [PES], einem sulfonierten Polyethersulfon [SPES], einem Polyphenylsulfon [PPSU], einem sulfonierten Polyphenylsulfon [SPPSU]; sowie Mischungen daraus; der polykationische Haftvermittler ein Polyethylenimin [PEI], beispielsweise mit einer Molekularmasse zwischen 1 kDa und 2 MDa ($M_w$), insbesondere ca. 2,0 kDa, ca. 25 kDa oder ca. 750 kDa ($M_w$), Chitosan, z.B. mit einer Molekularmasse zwischen 30 kDa und 750 kDa ($M_w$), Polylysin, z.B. mit einer Molekularmasse zwischen 15 kDa und 300 kDa ($M_w$) (z.B. Poly-L-Lysin Sigma), Polyarginin, z.B. mit einer Molekularmasse zwischen 1,9 kDa und 38,5 kDa ($M_w$) (beispielsweise n-butyl-poly-L-Argininhydrochlorid) oder Polyornithin, beispielsweise mit einer Molekularmasse zwischen 1,5 kDa und 30,1 kDa ($M_w$) ist; das Polyanion ein sulfatiertes Polysaccharid ist, welches ausgewählt ist aus: einem Dextransulfat [DEXS], beispielsweise mit einer Molekularmasse von 15 kDa bis 1 MDa ($M_w$), insbesondere ca. 500 kDa ($M_w$), einem sulfatierten Chitosan, z.B. mit einer Molekularmasse von 30 kDa bis 750 kDa ($M_w$); einem Cellulosesulfat, z.B. mit einer Molekularmasse zwischen 20 kDa und 1 MDa ($M_w$), vorzugsweise ca. 100 kDa ($M_w$); oder Mischungen daraus; und wobei das porenbildende hydrophile Additiv ausgewählt ist aus: Polyvinylpyrrolidon [PVP], z.B. mit einer Molekularmasse zwischen 1 kDa und 2,2 MDa ($M_w$), insbesondere ca. 1,1 MDa ($M_w$), einem kurzkettigen Glykol mit 2 bis 10 C-Atomen, Triethylenglykol, Propylenglykol, Polyethylenglykol [PEG]/Polyethylenoxid [PEO], beispielsweise mit einer Molekularmasse zwischen 600 Da und 500 kDa ($M_w$), insbesondere ca. 8 kDa ($M_w$) oder ca. 10 kDa ($M_n$); sowie Mischungen daraus.

Typischerweise weisen die erfindungsgemäß zum Einsatz kommenden Polysulfone folgende zahlenmittlere ($M_n$) und gewichtsgemittelte ($M_w$) Molekularmassenbereiche auf:

| | |
|---|---|
| Polysulfon [PSU]: | $M_n$ 16-22 kDa, $M_w$ 40-85 kDa, |
| sulfoniertes Polysulfon [SPSU]: | $M_n$ 27-32 kDa, $M_w$ 50-55 kDa, |
| Polyethersulfon [PES]: | $M_n$ 16-22 kDa, $M_w$ 30-75 kDa, |
| sulfoniertes Polyethersulfon [SPES]: | $M_n$ 18-22 kDa, $M_w$ 30-35 kDa, |
| Polyphenylsulfon [PPSU]: | $M_n$ 22 kDa, $M_w$ 52-55 kDa, und |
| sulfoniertes Polyphenylsulfon [SPPSU]: | $M_n$ 17-21 kDa, $M_w$ 47-53 kDa. |

Eine bevorzugte Ausführungsform der vorliegenden Erfindung ist eine solche Dialysemembran, bei welcher das SPSU einen Sulfonierungsgrad von 0,1 bis 20 Gew.-%, bezogen auf das Gewicht des unsulfonierten PSUs aufweist; und/oder das SPES einen Sulfonierungsgrad von 0,1 bis 20 Gew.-%, bezogen auf das Gewicht des unsulfonierten PES aufweist; und/oder das SPPSU einen Sulfonierungsgrad von 0,1 bis 20 Gew.-%, bezogen auf das Gewicht des unsulfonierten PPSUs aufweist.

Die Gewichtsprozentangaben werden für sulfoniertes SPES und SPSU aus NMR-Analysen berechnet. Die Proben wurden hierzu in deuteriertem Dimethylsulfoxid oder deuteriertem Chloroform gelöst und die NMR-Spektren wurden mit Hilfe eines 300 MHz oder eines 500 MHz NMR-Gerätes (Bruker) gemessen. Anhand der Ergebnisse, der molaren Verhältnisse zwischen nicht sulfonierten und sulfonierten Wiederholungseinheiten ($M_{PES}$ = 232 g/mol; $M_{SPES}$ = 312 g/mol) wurden die Sulfonierungsgrade von SPES3, SPES2 und SPES4 mit jeweils 1,1; 3,6 und 14,1 Gew.-% (Angabe Bereich von 0,1 bis 20 Gew.-%) ermittelt.

SPSUA, SPSUB mit 9,3 und 13,4 Gew.-% (Einheit: $M_{PSU}$ = 442 g/mol; $M_{SPSU}$ = 522 g/mol); Angabe Bereich von 0,1 bis 20 Gew.-%

Weiterhin, wird die Angabe für sPPSU auf den Massengehalt des sulfonierten Monomers 4,4'-Dichlordiphenylsulfon [sDCDPS] bezogen.

Im Rahmen der vorliegenden Erfindung können vorteilhafte Dialysemembranen hergestellt werden, wobei das SPSU einen Sulfonierungsgrad von 9,3 oder 13,4 Gew.-%, bezogen auf das Gewicht des unsulfonierten PSUs aufweist.

Ebenso sind solche Dialysemembranen bevorzugt, bei welchen das SPES einen Sulfonierungsgrad von 1,1; 3,6 oder 14,1 Gew.-%, bezogen auf das Gewicht des unsulfonierten PES aufweist.

In Bezug auf Dialysemembranen, welche SPPSU enthalten, sind solche bevorzugt, die einen Sulfonierungsgrad von 1,0, 2,0, 10,1 oder 14,7 Gew.-%, bezogen auf das Gewicht des unsulfonierten PPSUs aufweisen.

Als bevorzugte Additive haben sich Polyethylenglykol mit einer Molekularmasse von 600 Da bis 500 kDa ($M_w$), insbesondere ca. 8 kDa ($M_w$) oder 10 kDa ($M_n$) und/oder ein PVP mit einer Molekularmasse von 1 kDa bis 2,2 MDa ($M_w$), insbesondere ca. 1,1 MDa ($M_w$) herausgestellt.

Als bevorzugter Haftvermittler kann im Rahmen der vorliegenden Erfindung ein PEI mit einer Molekularmasse zwischen 1 kDa und 2 MDa ($M_w$), insbesondere ca. 2 kDa, bevorzugt ca. 25 kDa, vorzugsweise ca. 750 kDa ($M_w$) oder ein C2- bis C8-Dialkanal-vernetztes, insbesondere 1,5-Pentandial-vernetztes, hochmolekulares PEI eingesetzt werden. Insbesondere PEI mit einer Molmasse von 750 kDa ist kommerziell erhältlich, wogegen sich die eigens synthetisierten vernetzten PEI-Polymere hinsichtlich ihrer Eigenschaften, insbesondere deren Molekülgröße, z.B. über den Polymerisationsgrad und die Wahl des Vernetzers einstellen lassen.

Zur LbL-Beschichtung kann als Polysaccharid im Rahmen der vorliegenden Erfindung bevorzugt ein Dextransulfat mit einer Molekularmasse ($M_w$) von 15 kDa bis 1 MDa, insbesondere ca. 500 kDa und/oder ein sulfatiertes Chitosan mit einer Molekularmasse von 30 kDa bis 750 kDa; oder Mischungen daraus, verwendet werden.

Bei den bevorzugten Ausführungsformen der vorliegenden Erfindung kommen solche Dialysemembranen zum Einsatz, bei welchen das porenbildende Additiv ausgewählt ist aus: einem PVP mit einer Molekularmasse von ca. 1,1 MDa, Glycerin, einem PEG/Polyethylenoxid [PEO] mit einer Molekularmasse von 600 Da bis 500 kDa ($M_w$), insbesondere ca. 10 kDa ($M_n$) oder 8 KDa ($M_w$) oder einer 3-komponentigen Mischung daraus.

Die bevorzugten Dialysemembranen weisen eine Wasser-Permeabilität von 10 bis 2.000 L/bar*h*m$^2$ und einen Siebkoeffizienten (@22±2°C) für Rinderserumalbumin [BSA] im Bereich von 0,5 bis 0,0001 sowie einen Molekularmasse-Ausschlusswert von 20 bis 50 kDa auf.

Eine weitere bevorzugte Ausführungsform der erfindungsgemäßen Dialysemembran ist dadurch gekennzeichnet, dass das Polyethylenglykol/Polyethylenoxid eine Molekularmasse von 600 Da bis 500 kDa ($M_w$), insbesondere ca. 8 kDa ($M_w$) oder ca. 10 kDa ($M_n$), aufweist und/oder das PVP eine Molekularmasse von 1 kDa bis 2,2 MDa ($M_w$), insbesondere ca. 1,1 MDa ($M_w$) aufweist.

Eine weitere bevorzugte Ausführungsform der erfindungsgemäßen Dialysemembran ist dadurch gekennzeichnet, dass der Haftvermittler:

ein Polyethylenimin [PEI] mit einer Molekularmasse ($M_w$) von 1 kDa bis 2 MDa,
ein Chitosan mit einer Molekularmasse ($M_w$) von 40 bis 220 kDa,
ein Polylysin mit einer Molekularmasse ($M_w$) von 15 bis 300 kDa,
ein Polyarginin mit einer Molekularmasse ($M_w$) von 1,9 bis 38,5 kDa,
ein Polyornithin mit einer Molekularmasse ($M_w$) von 1,5 kDa bis 30,1 kDa, oder
eine Mischung daraus, ist.

Die zuvor genannte Dialysemembran ist ferner vorzugsweise dadurch gekennzeichnet, dass der Haftvermittler PEI eine Molekularmasse ($M_w$) von ca. 2 kDa, insbesondere ca. 25 kDa, vorzugsweise ca. 750 kDa, oder ein C2 bis C8-Dialkanalvernetztes, insbesondere 1,5-Pentandial-vernetztes, hochmolekulares PEI ist.

Eine weitere bevorzugte Ausführungsform der erfindungsgemäßen Dialysemembran ist dadurch gekennzeichnet, dass das Polysaccharid ein Dextransulfat mit einer Molekularmasse ($M_w$) von ca. 500 kDa und/oder ein sulfatiertes Chitosan mit einer Molekularmasse ($M_w$) von 30 kDa bis 750 kDa, ist.

Eine weitere bevorzugte Ausführungsform der erfindungsgemäßen Dialysemembran ist dadurch gekennzeichnet, dass das porenbildende Additiv ausgewählt ist aus: einem PVP mit einer Molekularmasse ($M_w$) von 1 kDa bis 2,2 MDa, insbesondere ca. 1,1 MDa, Glycerin, Propylenglykol, Triethylenglykol, einem PEG/PEO mit einer Molekularmasse(Mw) von 600 Da bis 500 kDa, insbesondere 8 bis 10 kDa oder einer mehrkomponentigen Mischung daraus.

Eine weitere bevorzugte Ausführungsform der erfindungsgemäßen Dialysemembran ist dadurch gekennzeichnet, dass der Kontaktwinkel einen Wert von 20 bis 70° aufweist und damit eine sehr gute Benetzbarkeit der Membranen (Hydrophilie) gewährleistet ist.

Die vorliegende Erfindung offenbart ebenfalls ein Verfahren zur Herstellung einer Dialysemembran in Hohlfasermembran- oder Flachmembran-Geometrie, wobei

a) man zur Herstellung einer Basismembran für die Dialysemembran eine Gieß- oder Spinnlösung aus wenigstens einem Polysulfon, welches ausgewählt ist aus: einem Polysulfon [PSU], einem sulfonierten Polysulfon [SPSU], einem Polyethersulfon [PES], einem sulfonierten Polyethersulfon [SPES], einem Polyphenylsulfon [PPSU], einem sulfonierten Polyphenylsulfon [SPPSU]; sowie Mischungen daraus; und wenigstens einem porenbildenden hydrophilen Additiv in wenigstens einem organischen Lösungsmittel, ausgewählt aus N,N-Dimethylacetamid, Dimethylsulfoxid, N-Methyl-2-pyrrolidon oder N-Ethyl-2-pyrrolidon herstellt; und

b) man die so hergestellte Polymermischungs-Lösung zur Ausbildung der Basismembran mit einem Fällungsmittel in Kontakt bringt, nach der Präzipitation der Polymermischung in Flach- oder Hohlfaserform das organische Lösungsmittel auswäscht; und

c) man die in Schritt b) hergestellte Basismembran zur Erzeugung einer Funktionalschicht darauf einer Oberflächenmodifikation unterzieht, indem man wenigstens eine Schicht-um-Schicht- [layer-by-layer, LbL] Abscheidung auf der Oberfläche der Basismembran durchführt, um die Dialysemembran zu erhalten, wobei man als erste Schicht auf der Oberfläche der Basismembran wenigstens einen polymeren polykationischen Haftvermittler aufbringt und auf der polykationischen Schicht als zweite Schicht wenigstens ein polymeres Polyanion aufbringt; und wobei

d) der polykationische Haftvermittler ausgewählt wird aus der Gruppe, bestehend aus Polyethylenimin [PEI] Chitosan, Polylysin, Polyarginin und Polyornithin, sowie Mischungen daraus ; und als Polyanion ein carboxyliertes Polysaccharid oder ein sulfatiertes Polysaccharid eingesetzt wird, welches ausgewählt wird, aus der Gruppe, bestehend aus: einem Dextransulfat mit einer Molekularmasse ($M_w$) von 15 kDa bis 300 MDa, einem sulfatierten Chitosan mit einer Molekularmasse ($M_w$) von 30 kDa bis 750 kDa; einem Cellulosesulfat mit einer Molekularmasse ($M_w$) zwischen 20 kDa und 1 MDa, vorzugsweise ca. 100 kDa; oder Mischungen daraus; und wobei das porenbildende hydrophile Additiv ausgewählt ist aus: Polyvinylpyrrolidon [PVP], einem kurzkettigen Glykol mit 2 bis 10 C-Atomen, Triethylenglykol, Propylenglykol, Polyethylenglykol [PEG]/Polyethylenoxid [PEO] sowie Mischungen daraus.

Für die Zwecke der vorliegenden Erfindung wird unter dem Begriff "Oberfläche der Basismembran" verstanden, dass sowohl die äußere als auch die Lumen-Oberfläche, umfasst ist.

Geeignete Fällungsmittel für Schritt b) in obigem Verfahren ist eine Mischung des organischen Lösungsmittels und Wasser mit einem Lösungsmittelanteil von 5 bis 85 Gew.-%.

Zur Herstellung nach dem erfindungsgemäßen Verfahren kann das Polyethylenglykol/Polyethylenoxid eine Molekularmasse ($M_w$) von 600 Da bis 500 kDa, insbesondere ca. 8 bis 10 kDa aufweisen und/oder das PVP mit einer Molekularmasse ($M_w$) von 1 kDa bis 2,2 MDa aufweisen.

Typischerweise kommt bei dem erfindungsgemäßen Verfahren PEI als Haftvermittler zum Einsatz, wobei ein PEI mit einer Molekularmasse von 1 kDa - 2 MDa, insbesondere ca. 750 kDa, oder ein C2 bis C8-Dialkanal-vernetztes, insbesondere 1,5-Pentandial-vernetztes, hochmolekulares PEI ist, vorzugsweise ein solches, welches aus einem PEI mit einer Molekularmasse von ca. 1,8 kDa durch Vernetzung mit 1,5-Pentandial herstellbar ist, bevorzugt ist.

Die bei dem erfindungsgemäßen Verfahren eingesetzten Polysaccharide sind vorzugsweise ein Dextransulfat mit einer Molekularmasse ($M_w$) von 15 kDa bis 1 MDa, insbesondre ca. 500 kDa und/oder ein sulfatiertes Chitosan mit einer Molekularmasse ($M_w$) von 30 kDa bis 750 kDa; oder Mischungen daraus.

Bei dem beschriebenen Verfahren wird das porenbildende Additiv ausgewählt aus: einem PVP mit einer Molekularmasse von ca. 1,1 MDa, Glycerin, einem PEG/PEO mit einer Molekularmasse von 600 Da bis 500 kDa, insbesondere 8 bis 10 kDa, oder einer 3-komponentigen Mischung daraus.

Es ist ferner bevorzugt, dass man den polymeren polykationischen Haftvermittler, insbesondere PEI, durch Aufsprühen auf die fertige Basismembran oder durch Zugabe zum Fällungsmittel auf die Oberfläche der Basismembran aufbringt.

Sowohl der polykationische Haftvermittler als auch die polyanionische Funktionsschicht können in besonders bevorzugter Weise durch Einspülen in das Lumen einer Kapillarmembran an einem Spülstand aufgebracht werden.

Eine weitere bevorzugte Ausführungsform des erfindungsgemäßen Verfahrens ist dadurch gekennzeichnet, dass die Komponenten der Gieß- oder Spinnlösung bei 20 bis 70 °C für 2 bis 18 h vollständig aufgelöst werden und die homogene Gieß- oder Spinnlösung zur Entfernung von Luftblasen bei 100 bis 800 mbar für 5 bis 45 min evakuiert wird.

Eine weitere bevorzugte Ausführungsform des erfindungsgemäßen Verfahrens ist dadurch gekennzeichnet, dass die Gieß- oder Spinnlösung eine Viskosität von 0,2 bis 5,0 Pa*s (@20 °C) aufweist.

Eine weitere bevorzugte Ausführungsform des erfindungsgemäßen Verfahrens ist dadurch gekennzeichnet, dass das porenbildende Additiv ausgewählt ist aus: einem PVP mit einer Molekularmasse ($M_w$) von 1,1 kDa bis 2,2 MDa, Glycerin, einem PEG/PEO mit einer Molekularmasse von 600 Da bis 500 kDa ($M_w$), insbesondere ca. 8 kDa ($M_w$) oder einer 3-komponentigen Mischung daraus.

Eine weitere bevorzugte Ausführungsform des erfindungsgemäßen Verfahrens ist dadurch gekennzeichnet, dass man den polymeren polykationischen Haftvermittler, insbesondere PEI, durch Aufsprühen auf die fertige Basismembran oder durch Zugabe zum Fällungsagens auf die Oberfläche der Basismembran oder durch Einspülen auf die Oberfläche der Basismembran an einem Spülstand aufbringt.

Eine weitere bevorzugte Ausführungsform des erfindungsgemäßen Verfahrens ist dadurch gekennzeichnet, dass der polymere polykationische Haftvermittler, insbesondere PEI, entsprechend einem Verhältnis von 1 bis 4000 $\mu$g /$cm^2$ Membranfläche aufgebracht wird.

Eine weitere bevorzugte Ausführungsform des erfindungsgemäßen Verfahrens ist dadurch gekennzeichnet, dass das polymere polyanionische Polysaccharid, insbesondere DEXS, entsprechend einem Verhältnis von 1 bis 1500 $\mu$g/$cm^2$ Membranfläche aufgebracht wird.

Eine weitere bevorzugte Ausführungsform des erfindungsgemäßen Verfahrens ist dadurch gekennzeichnet, dass das polymere polyanionische Polysaccharid, insbesondere DEXS, in einer 0,01 bis 1 M NaCl-Lösung aufgebracht wird.

Eine weitere bevorzugte Ausführungsform des erfindungsgemäßen Verfahrens ist dadurch gekennzeichnet, dass man das polymere polyanionische Polysaccharid, insbesondere DEXS, durch Einspülen auf die Lumenoberfläche der Basismembran an einem Spülstand aufbringt und somit die Trenneigenschaften der resultierenden Kompositmembran gezielt einstellt.

Eine weitere bevorzugte Ausführungsform des erfindungsgemäßen Verfahrens ist dadurch gekennzeichnet, dass der polymere polykationische Haftvermittler, insbesondere PEI, sowie das polymere polyanionische Polysaccharid, insbesondere DEXS, mit einem Transmembrandruck (TMP) von 10 bis 250 mbar und einer Beschichtungszeit von jeweils 2 bis 900 s aufgebracht wird.

Weitere Vorteile und Merkmale ergeben sich aufgrund der Beschreibung von Ausführungsbeispielen sowie anhand der Zeichnung.

Es zeigt:

Fig. 1    die Wasser-Permeabilität und Siebkoeffizienten von 5 unterschiedlichen Sets von Basismembranen vor ihrer LbL-Modifizierung;

Fig. 2    die Wirkung der LbL-Abscheidung auf die Wasser-Permeabilität und den Siebkoeffizienten für BSA bei 22±2 °C für die Polymeren der 5 unterschiedlichen Sets von Membranen;

Fig. 3    den Kontaktwinkel von nicht-modifizierten Membranen des Sets 1 als Maß für die Benetzbarkeit.;

Fig. 4    das Zetapotential für die Membranen des Sets 1 vor und nach LbL-Beschichtung

Fig. 5    die Molekülgrößenausschlussgrenze für Set 3, Beispiel P25, vor und nach LbL-Modifizierung vs. kommerzieller High-Flux xevonta B.Braun Membran;

Fig. 6    die Molmassenverteilung der polymeren Haftvermittler PEI 2 kDa sowie 750 kDa ($M_w$) und der Polysaccharide DEXS 15 kDa und 500 kDa, ermittelt mittels GPC.

I. Allgemeine Beschreibung der Herstellung von Basis-Membranen mit unterschiedlichen Polymeren.

Gieß- bzw. Spinnlösungen aus Basismembran-Polymer/Additiv/Lösungsmittel wurden wie folgt hergestellt: Zunächst wurde das ausgewählte Additiv und das gewünschte Polymer/Polymerblend in N,N-Dimethylacetamid [DMAc] und einer entsprechenden Wassermenge langsam aufgelöst (Lösung 1).

Als Membran-Polymere wurden folgende Verbindungen eingesetzt: Polysulfon [PSU] ($M_n$ 16-22 kDa, $M_w$ 40-85 kDa), sulfoniertes Polysulfon [SPSU] ($M_w$ 50-55 kDa, $M_n$ 27-32 kDa), Polyethersulfon [PES] ($M_w$ 30-75 kDa, $M_n$ 16-22 kDa), sulfoniertes Polyethersulfon [SPES] ($M_w$ 30-35 kDa, $M_n$ 18-22 kDa), Polyphenylsulfon [PPSU] ($M_w$ 52-55 kDa, $M_n$

22 kDa), sulfoniertes Polyphenylsulfon [SPPSU] ($M_w$ 47-53 kDa, $M_n$ 17-21 kDa); sowie Mischungen (Blends) daraus.

Die Modifizierung der Polymere PSU und PES wurde durch Postsulfonierung realisiert. Das Polymer wurde unter Schutzgas in Dichlormethan aufgelöst (Konzentration 3 bis 10 Gew.-%). Chlorsulfonsäure wurde als Reagenz benutzt. Es wurden 3 bis 10 g Polymer (PSU oder PES) eingesetzt. Die Menge von Chlorsulfonsäure (0,1 bis 20 ml) und Reaktionszeit (Zugabe innerhalb von 5 bis 45 Minuten; danach 1 bis 5 Stunden Reaktionszeit) wurden variiert, um den Sulfonierungsgrad einzustellen. Die Reaktion wurde bei Raumtemperatur durchgeführt. Danach wurde die Polymerlösung in eiskaltes Wasser als Fällbad überführt und das feste Polymer gewaschen bis ein pH-Wert von 6-7 erreicht wurde. Zuletzt wurde das modifizierte Polymer im Vakuumtrockenschrank bei 40 °C getrocknet.

Sulfoniertes Polyphenylsulfon [SPPSU] kann durch Copolymerisation hergestellt werden, gemäß Literatur, z.B. Wang et al.: Macromol. Symp. 175, 387-395 (2001) und/oder Dissertation von Jeffrey B. Mecham, Fakultät für Chemie des Virginia Polytechnic Institute and State University, Blacksburg, Virginia, USA, 23. April 2001.

Dabei wurden als Monomere kommerziell verfügbares 4,4'-Dihydroxybiphenyl (BP), 4,4'-Dichlordiphenylsulfon (DCDPS) sowie Di-Natrium-3,3'-di-sulfonat-4,4'-dichlordiphenylsulfon (sDCDPS) eingesetzt. Alle Reaktionen wurden in N-Methylpyrrolidon (NMP) durchgeführt. Als Base wurde Kaliumcarbonat verwendet.

Im Folgenden wird beispielhaft ein Herstellungsweg von sPPSU beschrieben:

In einem 2 l HWS-Kolben mit Rührer, Dean-Stark-Wasserabscheider, Stickstoffeinlass und Temperaturregelung wurden 258,37 g 4,4'-Dichlordiphenylsulfon (DCDPS), 186,21 g 4,4'-Dihydroxybiphenyl (BP), 49,12 g 3,3'-Dinatriumdisulfat-4,4'-dichlordiphenylsulfon und 146,5 g Kaliumcarbonat (mittlere Partikelgröße 50 $\mu$m) unter Stickstoffatmosphäre in 1000 ml NMP suspendiert. Die Mischung wurde unter Rühren bis auf 190°C erwärmt. Die Mischung wurde mit 20 l/h Stickstoff begast und 6h auf 190°C gehalten. Danach wurden 500 ml NMP zugegeben, um die Mischung abzukühlen. Man ließ die Mischung unter Stickstoff auf unter 60°C abkühlen. Nach Filtration wurde die Mischung in Wasser, welches 50 ml 2m HCl enthielt, ausgefällt. Das gefällte Produkt wurde 20 h bei 85°C mit heißem Wasser extrahiert und 24 h unter vermindertem Druck bei 120°C getrocknet.

Die Bestimmung der Molekulargewichtsverteilung erfolgte durch GPC unter Verwendung von DMAc/LiBr als Lösungsmittel und PMMA-Proben mit enger Molekulargewichtsverteilung als Standards, um das System zu kalibrieren.

Wie oben beschrieben, wurde das gewünschte Polymer/Polymerblend dann in N,N- Dimethylacetamid [DMAc] und einer entsprechenden Wassermenge langsam aufgelöst (Lösung 1). Diese Lösung 1 wurde kontinuierlich bei 40 °C gerührt, wenn Polyethylenglycol mit einem $M_n$ von 10 000 Da eingesetzt wurde. Falls PVP zum Einsatz kam, wurde bei 70 °C gerührt, wobei ein Poly(vinylpyrrolidon) mit einer gewichtsgemittelten Molmasse $M_w$ von ca. 1,1 MDa verwendet wurde. Um eine vollständige Auflösung der Polymerkomponenten zu erreichen, wurden die Mischungen wenigstens 3 bis 12 h gerührt. Falls zu diesem Zeitpunkt noch keine vollständige Auflösung erzielt wurde, wurde mehre Stunden weiter gerührt.

Die fertige homogene Gieß- bzw. Spinnlösung wurde bei 100 mbar für 15 min entgast, um Luftblasen zu entfernen.

Sofern nicht anders bezeichnet, sind sämtliche %-Angaben in Gew.-% angegeben und unter dem Begriff "Molmasse" wird, sofern nicht anders angegeben die gewichtsgemittelte Molmasse $M_w$ oder die zahlenmittlere Molmasse $M_n$ verstanden, welche beide mittels Gelpermeationschromatographie mit entsprechenden Molekularmassenstandards gemessen werden können. Der Grund hierfür liegt darin, dass die gewichtsgemittelte Molmasse für die Eigenschaftskorrelationen von Kunststoffen in der Praxis von größerer Bedeutung ist als die Zahlen- und Viskositätsmittelwerte (vgl. Saechtling, Kunststofftaschenbuch, 31. Ausgabe, Carl Hanser Verlag, München 2013).

Tabelle 1: Parameter für die Charakterisierung von Molmassen mittels GPC

| | PEI | DEXS | PSU, PES, PPSU, SPSU, SPES, SPPSU |
|---|---|---|---|
| Säule | NOVEMA 10000A, 10 $\mu$m (PSS) | SUPREMA 10000A, 10 $\mu$m (PSS) | GRAM Vorsäule + 2x GRAM, 10 $\mu$m (PSS) |
| Eluent | 0,1 M NaCl 0,25 % HCOOH 0,01 % NaN3 | 0,4 M NaCl 0,1 M NaNO3 0,01 % NaN3 | DMAc 0,01 M LiBr |
| Volumenstrom [ml/min] | | 1 | |
| Konzentration Polymer [g/L] | 2,5 | 1,0 | 4,0 |
| Detektor | RI | RI | ETA 2010 RI/VIS |

(fortgesetzt)

|  | PEI | DEXS | PSU, PES, PPSU, SPSU, SPES, SPPSU |
|---|---|---|---|
| Standard | PVP 900Da - 1MDa | Pullulan 180Da - 780kDa | PMMA 0,1-1000 kg/mol |

Zur Herstellung von Flachmembranen werden diese gemäß dem nicht-Lösungsmittelinduzierten Phasentrennverfahren [NIPS] hergestellt. Die Polymerlösung wird in einem Coatmaster 509 MC (Erichsen GmbH & CO. KG, Hemer) auf einer Glasplatte bei einer konstanten Rakelgeschwindigkeit von 25 mm/s mit einem Edelstahlrakel mit einer Spalthöhe von 200 $\mu$m zur Protomembran in einer kontrollierten Atmosphäre mit einer relativen Luftfeuchtigkeit < 30 % bei Raumtemperatur ausgezogen. Die Temperatur der Membran Herstellung - Gießlösung, Glasplatte, Edelstahlrakel, Fällbad - wurde von Raumtemperatur bis 60 °C variiert.

Der so erhaltene Gießfilm wird anschließend 5 min in ein Fällbad getaucht, welches als Fällmittel 500 ml einer Mischung 50 vol. % DMAc / 50 vol. % $H_2O$ enthält.

Nach der Präzipitation wird die Membran in Wasser überführt, wobei das Wasser 3 mal jeweils nach 20 min gewechselt wird. Für sogenannte "wet"-Varianten (vgl. Tabelle 2) werden die Membranen auf ihre Endgröße zugeschnitten und Proben bei Raumtemperatur in einer wässrigen Lösung von 10 mM Natriumazid bis zur Charakterisierung bzw. LbL-Modifikation aufbewahrt. Im Fall von getrockneten Varianten ("dry") werden die Membranen im Anschluss an das Waschen vor weiteren Schritten bei 100°C für 6 min getrocknet.

Zur Herstellung von Hohlfasermembranen wird die oben beschriebene fertige Polymer-Spinnlösung dem Ringspalt einer Hohlfadendüse, welche auf eine Temperatur von ca. 60°C temperiert ist, zugeführt. Gleichzeitig wird zur Ausbildung von Lumen und zur Induktion des Fällvorgangs eine Mischung von 50 vol. % DMAc / 50 vol. % $H_2O$ als Fällungsmittel durch die Düsennadel der Hohlfadendüse geführt. Die ausgeformte Hohlfasermembran wurde durch einen Kanal geführt, in welchem eine Temperatur von 50°C und eine relative Luftfeuchtigkeit von 90% herrschte, in etwa 70°C warmen Fällungsmittel (50 vol. % DMAc / 50 vol. % $H_2O$) ausgefällt, fixiert sowie anschließend gewaschen und aufbewahrt wie für die Flachmembranen beschrieben.

Nach dem Trocknen ergaben sich Hohlfasermembranen mit einem Lumendurchmesser von 200 $\mu$m und einer Wandstärke von 30 $\mu$m.

I.1.Modifikation der Basismembran: Layer-by-layer-Abscheidung

Die Oberflächenmodifikation der nach obiger Vorschrift hergestellten Flachmembranen wurde mittels einer Amicon® Ultrafiltrationsmesszelle oder einer in analoger Form und Abmessungen nachgebauten Zelle durchgeführt. Zunächst wurde eine Lösung eines kommerziell erhältlichen Polyethylenimins (750 kDa) entsprechend einem Verhältnis von 3,7 mg/cm$^2$ Membranfläche durch die Membran 5 min bei 50 mbar filtriert, um eine erste Schicht eines polymeren kationischen Haftvermittlers auf der Membranoberfläche aufzubringen. Dann wurde die Membranoberfläche mit Wasser gewaschen und Wasser wurde danach durch die Membran 5 min bei 50 mbar filtriert. Anschließend wurde eine 1 M NaCl-Lösung 2 min bei 50 mbar durch die Membran filtriert. Die Membran wurde danach mittels Filtration mit einer Dextransulfat-Lösung (DEXS-Lösung, 500 kDa in 1 M NaCl) entsprechend einem Verhältnis von 1,5 mg/cm$^2$ Membranfläche als zweite Schicht, welche das polymere Polyanion enthält, für 5 min bei 50 mbar beschichtet. Anschließend wurde die Membran wie zuvor beschrieben gewaschen, um überschüssiges Dextransulfat zu entfernen.

Bei Bedarf können die oben beschrieben LbL-Beschichtungen mehrfach wiederholt werden, um schlussendlich eine Funktionalschicht mit den gewünschten Eigenschaften zu erhalten.

Für die LbL-Beschichtung der im Rahmen der vorliegenden Erfindung hergestellten Hohlfasermembranen wurden die Hohlfasern zunächst in ein Dialysemodul eingefüllt, locker gepackt und dann die LbL-Beschichtung vorgenommen wie für die Flachmembranen beschrieben.

I.2.Permeabilitäts und Retentionsuntersuchungen

1.2.1. Charakterisierung der Basismembranen

Die Filtrationsexperimente wurden im sogenannten "dead-end-Konfigurationsmodus" unter Verwendung einer Amicon® Ultrafiltrationsmesszelle oder einer hauseigenen Zelle mit Rühreinrichtung durchgeführt. Für sämtliche Experimente wurde ein PP-Vlies unter die zu testenden Membranen gelegt, um zu verhindern, dass der Zellenboden die Membran mechanisch verändert.

Zunächst wurden die Membranen einer Kompaktierung unterzogen. Dies ist eine Druckwechsellast deren Effekte zu Veränderungen in der Membranstruktur und in Folge dessen zu einem Verlust der Wasser-Permeabilität führen. Die Membranen wurden zunächst durch Filtrationen von Reinstwasser bei 0,5 bar für wenigstens 30 min kompaktiert, bis ein quasi-konstanter Fluss erreicht wurde. Danach wurde der Druck für 10 min abgelassen und die Wasser-Permeabilität für ein Druckgefälle von 0,1 bis 0,5 bar gemessen. Die vorliegende Wasser-Permeabilität Lp, ausgedrückt in L $h^{-1}m^{-2}bar^{-1}$ wurde auf eine Temperatur von 20 °C standardisiert.

Der sich aufgrund der Kompaktierung ergebende Permeabilitätsverlust wurde gemäß folgender Gleichung 1 aus diesen Experimenten bestimmt:

$$L_{kp}(\cdots) \tag{1}$$

Die hergestellten Membranen wurden durch Rinderserumalbumin (BSA, Probumin, Millipore) mittels Ultrafiltration getestet. Zunächst wurden die zu testenden Membranen 2 Minuten durch Filtration einer Phosphatpufferlösung (8 g/L NaCl, 1,182 g/L $Na_2HPO_4$. $2H_2O$, 0,9 g/L $KH_2PO_4$) bei 50 mbar konditioniert. Anschließend wurde ein bekanntes Volumen einer BSA-Lösung als Feed mit einer Konzentration von 30 g/L BSA in Phosphatpuffer - entsprechend einem Verhältnis von 2,4 ml/cm² der Membran transmembran bei 30 mbar filtriert und Permeat- und Retentatproben im Verhältnis 1:5 gesammelt. Die Konzentrationen in der Feed-Lösung, Retentat und Permeat wurden bei 278,5-279,5 nm (nach entsprechender Kalibrierung) gemessen. Der Siebkoeffizient S für BSA wurde nach Gleichung 2 berechnet:

$$S = \frac{2 x Cperm\cdots}{Cfee dCrete\cdots} \tag{2}$$

$C_{permeat}$, $C_{feed}$ und $C_{retentat}$ sind die jeweiligen BSA-Konzentrationen in Permeat, Feed und Retentat.

Im Anschluss daran wurden die Membranen sorgfältig für 15 Minuten unter Rühren mit frischem Reinstwasser in der Amicon® Zelle gewaschen, um die schwach gebundene Fouling-Schicht zu entfernen. Danach wurde die Permeabilität erneut gemessen und der Fouling-Widerstand $R_f$ wie folgt berechnet:

$$R_f = \frac{Lp^*}{Lp} \tag{3}$$

worin Lp und Lp* die Permeabilitäten vor und nach BSA-Filtration sind.

Für die Zwecke der vorliegenden Anmeldung wird unter dem Begriff "Molekülgrößenausschlussgrenze (MWCO, molecular weight cut off) diejenige Molekülmasse verstanden, die durch eine Membran zu 90% zurückgehalten wird. Die Einheit ist Da.

Die Bestimmung der Molekülgrößenausschlussgrenze erfolgte mit einer Dextran-Breitbandmischung (Feed) mit einer Konzentration von 1,1 g/l (Massenverteilung/Liter: 0,20 g 1 kDa, 0,25 g 4 kDa, 0,15g 8 kDa, 0,07 g 15 kDa, 0,10 g 35 kDa, 0,15 g 70 kDa, 0,05 g 110 kDa, 0,13 g 250 kDa) in Wasser mit 0,01 % Natriumazid. Das Feed wurde entsprechend einem Verhältnis von 2,4 ml/cm² der Membran transmembran bei 30 mbar @ Raumtemperatur filtriert und Permeat- und Retentatproben im Verhältnis 1:5 gesammelt. Die Konzentrationen in der Feed-Lösung, Retentat und Permeat wurden anschließend mittels Gelpermeationschromatographie (PL-GPC 50 Plus, Varian) analysiert. Als Säule wurde eine PROTTEMA 300A (PSS), PL aquagel-OH Mixed 8$\mu$m (Agilent) verwendet und als Eluent wurde 0,01% $NaN_3$ in $H_2O$ mit einer Flussrate von 1 ml/min benutzt. Es wurden jeweils 100 $\mu$l der Proben injiziert. Die Auswertung erfolgte dabei auf Grundlage einer Kalibrierung mittels Polysaccharidstandard.

Mittels Software wird aus den Daten der Siebkoeffizient für jede Molmasse nach folgender Formel berechnet:

$$S = \frac{2 \cdot c_{Permeat}}{c_{Feed} + c_{Retentat}} \tag{4}$$

Durch Darstellung des Siebkoeffizienten in Abhängigkeit der Molmasse (logarithmische Auftragung) wird die Siebkurve für die jeweilige Membran erhalten. Aus der Siebkurve wird dann der Cut-Off für einen Siebkoeffizienten von 0,1 ermittelt. Unter dem Begriff "Molekülgrößenausschlussgrenze" (MWCO, molecular weight cut off) wird diejenige Molekülmasse verstanden, die durch eine Membran zu 90% zurückgehalten wird. Die Einheit ist Dalton [Da].

I.2.2. Charakterisierung der LbL-modifizierten Membranen

Zur Charakterisierung der LbL-modifizierten Membranen wurden die Messungen wie für die Basismembranen oben beschrieben in analoger Weise durchgeführt. Zunächst wurden die unmodifizierte Membran kompaktiert und die Permeabilität gemessen (bei einem Druckgefälle von 0,1 bis 0,5 bar). Anschließend wurde die LbL-Beschichtung durchgeführt wie in Abschnitt I.2. beschrieben. Im Anschluss an die LbL-Beschichtung wurde die neue Permeabilität gemessen und der durch die LbL-Abscheidung bedingte Permeabilitätsverlust $Lp_{LbL\text{-Verlust}}$ gemäß Gleichung 4 berechnet:

$$L_{p\,LbL} = \frac{Q_{LbL} \cdot f \cdot l}{p \cdot A \cdot T_c} \quad (5)$$

Die neue Membranleistungsfähigkeit wurde ebenfalls durch BSA-Rückhalt gemessen, unter Anwendung desselben Verfahrens wie für die Basismembranen. Schlussendlich wurden die Membranen wiederum sorgfältig für 15 Minuten unter Rühren mit Reinstwasser gewaschen und dann erneut 5 Minuten bei einem Druck von 0,1 bar gespült. Danach wurde wiederum die Permeabilität gemessen und der MWCO bzw. Fouling-Widerstand $R_{f\text{-LbL}}$ ermittelt.

I.3. Kontaktwinkelmessungen

Der Kontaktwinkel in "°" wurde unter Verwendung einer optischen Kontaktwinkel-Messvorrichtung bestimmt. Die Messungen wurden unter Verwendung eines statischen "Captive-Bubble"-Verfahrens (Luftblasenvolumen: 5 $\mu$l). Für jede Probe wurden mindestens 5 Messungen an unterschiedlichen Positionen durchgeführt und dann der Mittelwert berechnet.

I.4. Zetapotentialmessungen

Zur Bestimmung der Oberflächenladung der Lumenoberfläche der hergestellten Membranen wurde das Zetapotential mit einem kommerziellen elektrokinetischen SurPASS Messgerät (Anton Paar) bestimmt. Vor dem jeweiligen Versuch wurden die zu messenden Membranen 1 Stunde in einer Elektrolytlösung aus 1 mM KCl äquilibriert. Die Versuche wurden dann bei Raumtemperatur von einem pH-Wert von 3, eingestellt mit einer HCl-Lösung, bis zu einem schrittweise durch Zugabe einer KOH-Lösung erhöhten pH-Wert auf einen pH-Endwert von 11,5 durchgeführt.

I.5. Rheologische Messungen

Die Viskosität der hergestellten Gieß- bzw. Spinnlösungen wurde unter Verwendung eines Rheometers, das mit einem Peltier-Element zur Temperatursteuerung ausgestattet ist, gemessen. Die Versuche wurden bei einer konstanten Scherrate (125 1/s) bei einem Temperaturgefälle (zwischen 20 und 60 °C) unter Verwendung eines Messsystems mit einer verjüngten Platte (CP25-2 / TG) durchgeführt

II. Ergebnisse

Die Zusammensetzung der Gießlösungen zur Herstellung von Flachmembranen als Modellmembranen sowie die Herstellungstemperatur sind in Tabelle 2 dargestellt.

In der Tabelle ist PSU Polysulfon, SPPSU1, SPPSU2, SPPSU10 , SPPSU14 entspricht den jeweiligen angegebenen Sulfonierungsgraden der eingesetzten sulfonierten Polyphenylsulfone in Gew.-%, demnach liegen für die zuvor angegebenen sulfonierten Polyphenylsulfone folgende Sulfonierungsgrade vor: 1,1; 3,6 und 14,1 Gew.-%.

Für die eingesetzten sulfonierten Polysulfonether gilt für ihren jeweiligen Sulfonierungsgrad in Analogie zu den oben für die sulfonierten Polyphenylsulfone dargelegte Nomenklatur jeweils dasselbe für die verwendeten sulfonierten Polyethersulfone SPES3 , SPES2 und SPES4. Diese sulfonierten Polyethersulfone weisen somit jeweils einen Sulfonierungsgrad von 1,1; 3,6 und 14,1 Gew.-% auf.

Für die Versuche wurden 5 verschiedenen Sets an Gießlösungen gewählt:

- Set 1 : 15 % Polymergehalt in der Gießlösung unter Verwendung unterschiedlicher Anteile an SPPSU und PVP als Additiv.

- Set 2 : 17 % Polymergehalt in der Gießlösung unter Verwendung unterschiedlicher Anteile an SPPSU und PVP als Additiv.

- Set 3 : 16 % Polymergehalt in der Gießlösung unter Verwendung unterschiedlicher Anteile an SPPSU und PEG/PEO als Additiv.

- Set 4 : 15 % Polymergehalt in der Gießlösung unter Verwendung unterschiedlicher Anteile an SPES und PVP als Additiv.

Eine Zusammenfassung der für die getesteten Membranen erhaltenen experimentellen Daten ist in Tabelle 3 dargestellt.

**Tabelle 2**: Zusammensetzungen der für die Membranherstellung eingesetzten Lösungen und Gießbedingungen

| | Set 1 | Set 1 | Set 1 | Set 1 | Set 1 | Set 2 | Set 2 |
|---|---|---|---|---|---|---|---|
| Polymerlösungen | SO-PVP-B | S1-PVP-B | S2-PVP-B | S10-PVP-B | S14-PVP-B | S2-PVP-D | S10-PVP-5D |
| PSU/SPPSU* | 15/0 | 13,5/1,5 | 13,5/1,5 | 13,5/1,5 | 13,5/1,5 | 15,3/1,7 | 16,2/0,8 |
| PVP/DMA$_C$/H$_2$O | 3,7/80,8/0,5 | 3,7/80,8/0,5 | 3,7/80,8/0,5 | 3,7/80,8/0,5 | 3,7/80,8/0,5 | 3,7/78,8/0,5 | 3,7/78,8/0,5 |
| Herstellungstemp. (°C) | 60 | 60 | 60 | 60 | 60 | 60 | 60 |

| | Set 3 | Set 3 | | Set 3 | Set 3 | Set 3 |
|---|---|---|---|---|---|---|
| Polymerlösungen | S2-PEG-A | S2-PEG-A-25 | | S10-PEG-A | S14-PEG-A | P25 |
| PSU/SPPSU* | 14,4/1,6 | 14,4/1,6 | | 14,4/1,6 | 14,4/1,6 | |
| PEG/DMAc | 5/79 | 5/79 | | 5/79 | 5/79 | |
| PSU/PEG/DMAc | | | | | | 16/5/79 |
| Herstellungstemp. (°C) | 40 | 25 | | 40 | 40 | 23±3 |

| | Set 4 | Set 4 | | Set 4 | Set 5 | |
|---|---|---|---|---|---|---|
| Polymerlösungen | SPES2-PVP-B | SPES3-PVP-B | | SPES4-PVP-B | P29 | |
| PSU/SPES | 13,5/1,5 | 13,5/1,5 | | 13,5/1,5 | | |
| PVP/DMAc/H$_2$0 | 3,7/80,8/0,5 | 3,7/80,8/0,5 | | 3,7/80,8/0,5 | | |
| PPSU/PEG/DMAc | | | | | 14,3/5,1/80,6 | |
| Herstellungstemperatur (°C) | 60 | 60 | | 60 | 23±3 | |

*Die verwendeten sulfonierten Polymere sind im Namen der Polymerlösung enthalten S1, S2, S10 und S14 entsprechen jeweils, SPPSU1, SPPSU2, SPPSU10, SPPSU14.

Tabelle 3: Zusammenfassung der experimentellen Daten der getesteten Membranen auf

| Polymerlösung** | S0-PVP-B | S1-PVP-B | S2-PVP-B | S10-PVP-B | S14-PVP-B |
|---|---|---|---|---|---|
| Zustand Membran | wet | wet | wet | wet | wet |
| Lp vor Kompaktierung @0.5 bar (L h$^{-1}$m$^{-2}$bar$^{-1}$) | 674 ± 78[6] | 746 ± 21[3] | 741 ± 121[7] | 1432 ± 137[5] | 1720 ± 446[8] |
| Lp nach Kompaktierung @0.5 bar (L h$^{-1}$m$^{-2}$bar$^{-1}$) | 578 ± 31[6] | 709 ± 16[3] | 675 ± 122[7] | 1376 ± 161[5] | 1505 ± 482[8] |
| Lp Kompaktierungsverlust (%) | 13 ± 10[6] | 2 ± 2[3] | 9 ± 5[7] | 4 ± 2[5] | 14 ± 8[8] |
| **Lp (L h$^{-1}$m$^{-2}$bar$^{-1}$)** | 509 ± 55[6] | 652 ± 16[3] | 597 ± 92[7] | 1231 ± 204[5] | 1292 ± 412[8] |
| Lp BSA (L h$^{-1}$m$^{-2}$bar$^{-1}$) | 105 ± 9[3] | 138 ± 8[3] | 83 | 207 ± 26[3] | 278 ± 76[4] |
| Lp after BSA (L h$^{-1}$m$^{-2}$bar$^{-1}$) | 239 ± 37[3] | 277 ± 37[3] | 268 | 274 ± 20[3] | 349 ± 54[4] |
| Fouling Widerstand R$_f$ (-) | 0,44 ± 0,03[3] | 0,42 ± 0,05[3] | 0,61 | 0,24 ± 0,03[3] | 0,36 ± 0,07[4] |
| **Siebkoeffizient** | 0,063 ± 0,065[3] | 0,054 ± 0,017[3] | 0,020 ± 0,002[3] | 0,085 ± 0,044[3] | 0,154 ± 0,086[4] |

**Die verwendeten sulfonierten Polymere sind im Namen der Polymerlösung enthalten S1, S2, S10 und S14 entsprechen jeweils SPPSU1, SPPSU2, SPPSU10, SPPSU14

EP 3 222 341 A1

**Tabelle 3** (Fortsetzung)

| Polymerlösung** | S0-PVP-B | S1-PVP-B | S2-PVP-B | S10-PVP-B | S14-PVP-B |
|---|---|---|---|---|---|
| **Layer by Layer** | | | | | |
| Lp LbL @0.5 bar (L $h^{-1}m^{-2}bar^{-1}$) | $433 \pm 25^3$ | - | $432 \pm 49^3$ | $693^{\,2}$ | $546^{\,2}$ |
| **Lp LbL (L $h^{-1}m^{-2}bar^{-1}$)** | $408 \pm 22^3$ | - | $406 \pm 46^3$ | $709^{\,2}$ | $514^{\,2}$ |
| Lp Verlust nach LbL (%) | $15 \pm 9^3$ | - | $39 \pm 4^3$ | $45^{\,2}$ | $62^{\,2}$ |
| Lp BSA (L $h^{-1}m^{-2}bar^{-1}$) | $128 \pm 23^3$ | - | $99 \pm 7^3$ | $153^{\,2}$ | $120^{\,2}$ |
| Lp nach BSA (L $h^{-1}m^{-2}bar^{-1}$) | $174 \pm 26^3$ | - | $164 \pm 9^3$ | $319^{\,2}$ | $255^{\,2}$ |
| Fouling-Widerstand $R_{f\text{-}LbL}$ (-) | $0{,}43 \pm 0{,}05^3$ | - | $0{,}41 \pm 0{,}04^3$ | $0{,}45^{\,2}$ | $0{,}50^{\,2}$ |
| **Siebkoeffizient** | $0{,}005 \pm 0{,}002^3$ | - | $0{,}005 \pm 0{,}002^3$ | $0{,}023^{\,2}$ | $0{,}017^{\,2}$ |
| **Charakterisierung** | | | | | |
| Kontaktwinkel (°) | $47 \pm 1$ | $34 \pm 2$ | $39 \pm 1$ | $38 \pm 3$ | $41 \pm 4$ |
| Viskosität (Pa.s, 20 °C) | 3,4 | 3,4 | 3 | 2,7 | 2,3 |

**Die verwendeten sulfonierten Polymere sind im Namen der Polymerlösung enthalten S1, S2, S10 und S14 entsprechen jeweils SPPSU1, SPPSU2, SPPSU10, SPPSU14

**Tabelle 3** (Fortsetzung)

| Polymerlösung | SPES3-PVP-B | SPES2-PVP-B | SPES4-PVP-B | S2-PVP-D | S10-PVP-5D |
|---|---|---|---|---|---|
| **Zustand Membran** | **wet** | **wet** | **wet** | **wet** | **wet** |
| Lp vor Kompaktierung @0.5 bar (L $h^{-1}m^{-2}bar^{-1}$) | $713 \pm 126^5$ | $744 \pm 49^4$ | $1335 \pm 256^3$ | $631 \pm 81^7$ | $931 \pm 214^5$ |
| Lp nach Kompaktierung @0.5 bar (L $h^{-1}m^{-2}bar^{-1}$) | $625 \pm 125^5$ | $659 \pm 66^4$ | $1292 \pm 228^3$ | $564 \pm 76^7$ | $891 \pm 202^5$ |
| Lp Kompaktierungsverlust (%) | $13 \pm 5^5$ | $11 \pm 6^4$ | $3 \pm 2^3$ | $10 \pm 7^7$ | $4 \pm 1^5$ |
| **Lp (L $h^{-1}m^{-2}bar^{-1}$)** | $593 \pm 104^5$ | $630 \pm 45^4$ | $1206 \pm 176^3$ | $533 \pm 97^7$ | $774 \pm 159^5$ |
| Lp BSA (L $h^{-1}m^{-2}bar^{-1}$) | $117 \pm 21^3$ | $154 \pm 30^3$ | 208 | $154 \pm 13^4$ | $397 \pm 97^3$ |
| Lp nach BSA (L $h^{-1}m^{-2}bar^{-1}$) | $243 \pm 16^3$ | $339 \pm 30^3$ | 247 | $193 \pm 17^4$ | $254 \pm 34^3$ |
| Fouling-Widerstand $R_f$ (-) | $0,48 \pm 0,08^3$ | $0,52 \pm 0,06^3$ | 0,21 | $0,32 \pm 0,02^4$ | $0,32 \pm 0,04^3$ |
| **Siebkoeffizient** | $0,146 \pm 0,071^3$ | $0,360 \pm 0,197^3$ | 0,893 | $0,067 \pm 0,056^4$ | $0,063 \pm 0,032^3$ |
| **Layer by Layer** | | | | | |
| Lp LbL @0.5 bar (L $h^{-1}m^{-2}bar^{-1}$) | $520^2$ | 455 | $768^2$ | $287 \pm 74^3$ | $487^2$ |
| **Lp LbL (L $h^{-1}m^{-2}bar^{-1}$)** | $491^2$ | 463 | $739^2$ | $266 \pm 72^3$ | $457^2$ |
| LP Verlust nach LbL (%) | $30^2$ | 16 | $38^2$ | $39 \pm 13^3$ | $35^2$ |
| Lp BSA (L $h^{-1}m^{-2}bar^{-1}$) | $124^2$ | 88 | $233^2$ | $103 \pm 13^3$ | $105^2$ |
| Lp nach BSA (L $h^{-1}m^{-2}bar^{-1}$) | $183^2$ | 242 | $307^2$ | $124 \pm 18^3$ | $166^2$ |
| Fouling-Widerstand $R_{f\text{-}LbL}$ (-) | $0,37^2$ | 0,52 | $0,41^2$ | $0,49 \pm 0,08^3$ | $0,36^2$ |
| **Tabelle 3** (Fortsetzung) | $0,030^2$ | 0,047 | $0,219^2$ | $0,002^2$ | $0,002^2$ |

**Siebkoeffizient**

| Polymerlösung | SPES3-PVP-B | SPES2-PVP-B | SPES4-PVP-B | S2-PVP-D | S10-PVP-5D |
|---|---|---|---|---|---|
| **Charakterisierung** | | | | | |
| Kontaktwinkel (°) | 35 ± 1 | 36 ± 3 | 44 ± 6 | 39 ± 5 | 44 ± 6 |
| Viskosität (Pa.s, 20 °C) | 2,3 | 2,6 | 2,2 | 4,4 | 4,2 |

**Tabelle 3** (Fortsetzung)

| Polymerlösung | S2-PEG-A | S10-PEG-A | S14-PEG-A | S2-PEG-A-25 |
|---|---|---|---|---|
| Zustand Membran | wet | wet | wet | wet |
| Lp vor Kompaktierung @0.5 bar (L $h^{-1}m^{-2}bar^{-1}$) | 2723 ± 335[6] | 3063 ± 219[6] | 3625 ± 340[3] | 2670 |
| Lp nach Kompaktierung @0.5 bar (L $h^{-1}m^{-2}bar^{-1}$) | 2507 ± 273[6] | 2824 ± 159[6] | 2888 ± 318[3] | 2316 |
| Lp Kompaktierungsverlust (%) | 8 ± 4[6] | 8 ± 4[6] | 20 ± 9[3] | 13 |
| **Lp (L $h^{-1}m^{-2}bar^{-1}$)** | 2266 ± 286[6] | 2272 ± 114[6] | 2651 ± 260[3] | 2126 |
| Lp BSA (L $h^{-1}m^{-2}bar^{-1}$) | 535 ± 64[3] | 478 ± 22[3] | 720 | - |
| Lp nach BSA (L $h^{-1}m^{-2}bar^{-1}$) | 805 ± 132[3] | 921 ± 68[3] | 1181 | - |
| Fouling-Widerstand $R_f$ (-) | 0,38 ± 0,02[3] | 0,42 ± 0,03[3] | 0,49 | - |
| **Siebkoeffizient** | 0,193 ± 0,055[3] | 0,293 ± 0,036[3] | 0,647 | - |
| **Layer by Layer** | | | | |
| Lp LbL @0.5 bar (L $h^{-1}m^{-2}bar^{-1}$) | 1142 ± 166[3] | 853 ± 36[3] | 1270 [2] | 1042 |
| **Lp LbL (L $h^{-1}m^{-2}bar^{-1}$)** | 1102 ± 144[3] | 793 ± 26[3] | 1220 [2] | 974 |
| LP-Verlust nach LbL (%) | 54 ± 5[3] | 66 ± 1[3] | 56 [2] | 54 |
| Lp BSA (L $h^{-1}m^{-2}bar^{-1}$) | 161 ± 39[3] | 283 ± 15[3] | 215 [2] | 186 |
| Lp nach BSA (L $h^{-1}m^{-2}bar^{-1}$) | 448 | 384 ± 28[3] | 526 | - |
| Fouling-Widerstand $R_{f-LbL}$ (-) | 0,34 | 0,48 ± 0,02[3] | 0,42 | - |
| **Siebkoeffizient** | 0,012 ± 0,004[3] | 0,006 ± 0,003[3] | 0,032 [2] | 0,007 |
| **Charakterisierung** | | | | |
| Kontaktwinkel (°) | - | - | - | - |
| Viskosität (Pa.s, 20 °C) | 0,8 | 0,7 | 0,7 | 0,8 |

**Tabelle 3** (Fortsetzung)

| Polymerlösung | P25* | P29* | P29_limA** | P29_limB** | P29 limB** |
|---|---|---|---|---|---|
| Zustand Membran | wet | dry | dry | dry | wet |
| Lp vor Kompaktierung @0.5 bar (L h⁻¹m⁻²bar⁻¹) | 2423±3 25[18] | 1208±25 2[27] | 1208±252[27] | 1208±252 27 | 3638±1241[4] |
| Lp nach Kompaktierung @0.5 bar (L h⁻¹m⁻²bar⁻¹) | 1899±3 20[18] | 690±284 27 | 690±284[27] | 690±284[27] | 1985±298[4] |
| Lp Kompaktierungsverlust (%) | 21±10[18] | 44±18[27] | 44±18[27] | 44±18[27] | 56±29[4] |
| **Lp (L h⁻¹m⁻²bar⁻¹)** | - | - | - | - | - |
| Lp BSA (L h⁻¹m⁻²bar⁻¹) | - | - | - | - | 356[1] |
| Lp nach BSA (L h⁻¹m⁻²bar⁻¹) | - | 300± 80[4] | 300± 80[4] | 300± 80[4] | 1103[1] |
| Fouling-Widerstand Rf (-) | - | 0.69±0.09[4] | 0.69±0.09[4] | 0.69±0.09 4 | 0,48[1] |
| **Siebkoeffizient** | 0.0640[2] | 0.247 ± 0.078[5] | 0.247 ± 0.078[5] | 0.247 ± 0.078[5] | 0,3542[1] |
| **MWCO (kDa)** | 113±61[8] | 97±36[2] | 97±36[2] | 97±36[2] | 122[1] |
| **Layer by Layer** | | | | | |
| **Lp LbL (L h⁻¹m⁻²bar⁻¹)** | 720±19 3[11] | 65±22[3] | 228±39[3] | 138±82[9] | 456[2] |
| LP Verlust nach LbL (%) | 58±12 | 84±11[3] | 64±15[3] | 79±15[9] | 71[2] |
| Lp BSA (L h⁻¹m⁻²bar⁻¹) | 167±32 7 | - | 60[1] | 36±16[3] | 113[2] |
| Lp nach BSA (L h⁻¹m⁻²bar⁻¹) | 428±11 7[7] | - | 147[1] | 107±41[3] | 118[2] |
| Fouling-Widerstand Rf-LbL (-) | - | - | 0,65[1] | 0,56±0,12 3 | 0,26[2] |
| **Siebkoeffizient** | 0.0013 ±0,000 5[7] | - | 0,0083[1] | 0,0045±0, 0009[3] | 0,01215[2] |
| **MWCO (kDa)** | 34±2[5] | 29[1] | 50[2] | - | - |

* LbL-Modifizierung erfolgte entsprechend I.1

** LbL-Modifizierung mit limitierten Mengen Polyelektrolyt: 112 µg/cm² PEI (750 kDa, 50 mbar, 5 min), 21 µg/cm² DEXS (500 kDa, 50 mbar, 5 min) für limA; 3,7 mg/cm² PEI (750 kDa, 50 mbar, 5 min), 21 µg/cm² DEXS (500 kDa, 50 mbar, 5 min) für limB

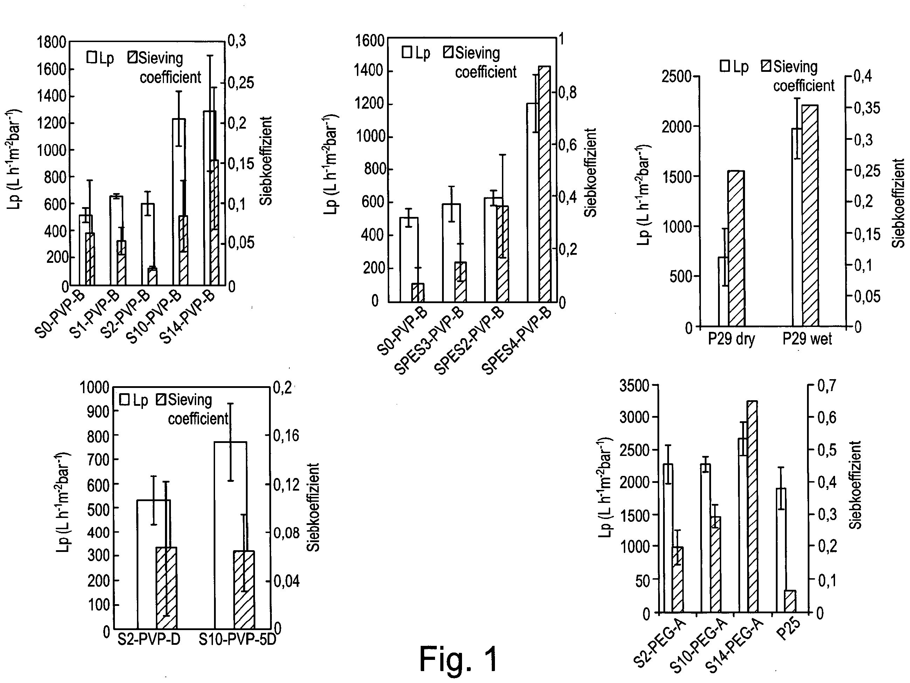

Zur besseren Anschaulichkeit der Eigenschaften der erfindungsgemäßen Membranen sind in Fig. 1 die gemessenen Wasserpermeabilitäten und Siebkoeffizienten vor der Modifikation von 5 verschiedenen Membrantypen gezeigt (vgl. Tabelle 2 und 3). Die hydraulischen Permeabilitäten der Basismembranen liegen zwischen 10 und 5.000 L/bar*h*m$^2$ und weisen einen Siebkoeffizienten (@22$\pm$2°C) für BSA im Bereich von 0,9 bis 0,001 sowie einen MWCO von 3 kDa bis 250 kDa auf. Im Falle der Membranen mit mittelmäßigem Flux, welche Membranen mit PVP als Additiv entsprechen, zeigen die Ergebnisse deutlich , dass die Verwendung von sulfonierten Polymeren zu einer drastischen Erhöhung der Wasserpermeabilität führen, was vermutlich hauptsächlich auf eine höhere Membranhydrophilie derartiger Membranen zurückzuführen ist.

Für die High-Flux-Membranen - entsprechend den Membranen mit PEG/PEO als Additiv - ist der Effekt jedoch weniger ausgeprägt. Dennoch werden in sämtlichen Fällen hohe Permeabilitäten auf Kosten der Größenrückhaltungseigenschaften erhalten. Ein vermutlicher Anstieg der Porengrößen ist wahrscheinlich der Grund hierfür. Niedrigere Viskositäten werden für solche Gießlösungen erhalten, die mit Polymeren mit einem höheren Sulfonierungsgrad hergestellt wurden. Dies induziert eine Änderung in der Membranbildung während des Phaseninversionsprozesses.

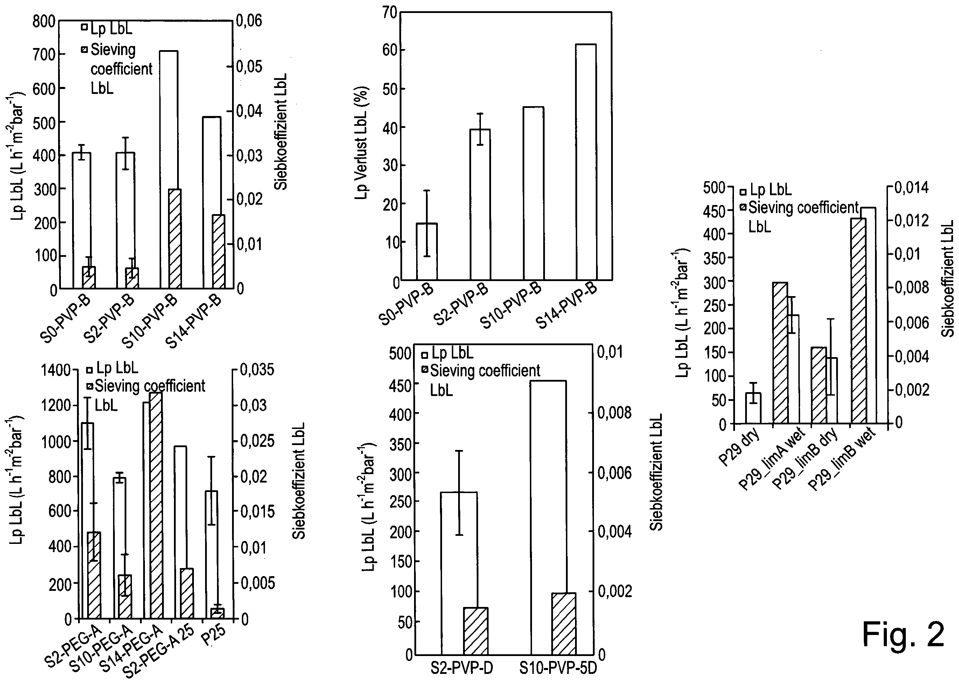

Figur 2 zeigt die Wirkung der LbL-Abscheidung auf Wasser-Permeabilität und BSA-Siebkoeffizienten für 4 unterschiedliche Sets von Membranen, sowie die durch LbL-Abscheidung induzierte Permeabilitätsverringerung.

Nach LbL-Beschichtung mit den Polyelektrolyten wird ein Abfall der Permeabilität auf bis zu 84% beobachtet. Insbesondere für das Set 1 mit PVP als Additiv wird eine direkte Korrelation zwischen dem Grad der Sulfonierung des Polymers und der Permeabilität beobachtet; die Verwendung eines sulfonierten Polymers mit einem höheren Sulfonierungsgrad in der Mischungslösung führt zu einer größeren Verringerung der Permeabilität durch die Beschichtung.

Ohne hieran gebunden zu sein, könnte einerseits die Gegenwart einer höheren Ladungsdichte an der Membranoberfläche zu einer besseren Haftung der Polyelektrolyten während der Abscheidung führen. Andererseits könnte dieselbe Menge an Polyelektrolyten abgeschieden worden sein und die größere Permeabilitätsverminderung könnte durch das Vorliegen größerer Poren zu Beginn des LbL-Prozesses verursacht sein, wobei die Poren während der Abscheidung der Polyelektrolyte durch die Modifizierung verengt oder blockiert worden sein könnten.

Ferner zeigt Fig. 2, dass hohe Permeabilitäten und niedrige Siebkoeffizienten für die mit PEG/PEO als Additiv hergestellten Membranen nach der Modifizierung erhalten werden.

S2-PEG-A, S2-PEG-A25, S10-PEG-A und P25 zeigen jeweils Wasser-Permeabilitäten von 1102 $\pm$ 144 L*bar*h-1*m-2, 974 L*bar*h-1*m-2, 793 $\pm$ 26 L*bar*h-1*m-2, 720 $\pm$ 193 L*bar*h-1*m-2 und Siebkoeffizienten für BSA bei 22 $\pm$ 2°C von 0,012 $\pm$ 0,004, 0,007, 0,006 $\pm$ 0,003 und 0,0013 $\pm$ 0,0005.

Erfindungsgemäß modifizierte Membranen aus Set 2, welche aus einer Polymerlösung, die 17% Polymer und PVP als Additiv enthalten - zeigen eine hohe Leistung. Es wurden Wasser-Permeabilitäten von 266 $\pm$ 72 L*bar*h-1*m-2 und 457 L*bar*h-1*m-2 erreicht und BSA-Siebkoeffizienten von jeweils 0,002 wurden für S2-PVP-D und S10-PVP-5D erhalten.

Kompositmembranen aus Set 4, welche aus einer Polymerlösung mit 14,3 % Polymer und PEG als Additiv hergestellt sind, zeigen in Abhängigkeit der Beschichtungsmenge und den Bedingungen bei der Modifizierung Wasser-Permeabilitäten von 65 $\pm$ 22 bis 456 L*bar*h-1*m-2 und BSA-Siebkoeffizienten von 0,0083 bis 0,01215.

Als Ergebnis wurde eine breite Palette von Membranen erhalten, welche eine mittlere bis hohe Wasserpermeabilität besitzen. Desweiteren zeigen die Ergebnisse, dass die LbL-Beschichtung den dominierenden Einfluss auf die Wasser-Permeabilität und den Siebkoeffizienten für BSA haben, so dass die durch LbL-Beschichtung erhaltenen Membranen für die Dialyseanwendung geeignet sind.

Insbesondere konnte im Rahmen der vorliegenden Erfindung durch Einstellen des Gehalts und des Sulfonierungsgrades des verwendeten Polymers eine Kontrolle über die Membranoberflächenladung erreicht werden.

Im Rahmen der vorliegenden Erfindung können somit zur Reinigung von Blut (bzw. Dialyse) geeignete Membranen hergestellt werden, die eine Wasser-Permeabilität im Bereich von 10 bis 2000 L*bar*h-1*m-2 und einem Siebkoeffizienten @ 22 $\pm$ 2°C für BSA im Bereich von 0,0001 bis 0,5 aufweisen.

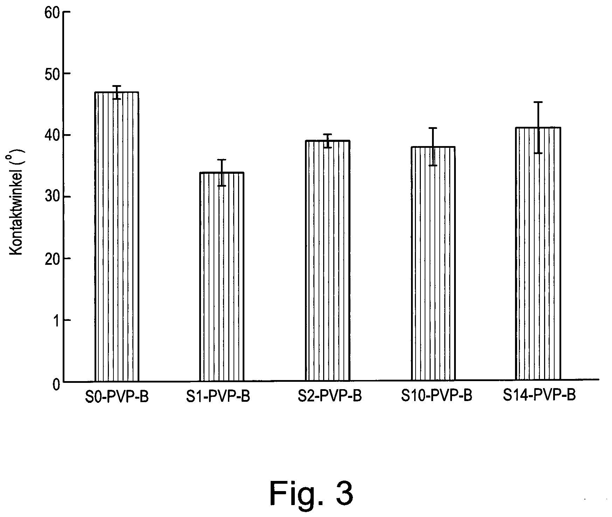

Eine ergänzende Analyse hinsichtlich der Hydrophilie der Membranen (insbesondere Set 1) ist in Fig. 3 dargestellt: Diese zeigt Kontaktwinkelmessungen an einer PSU/PVP-Basismembran (S0-PVP-B) und an Membranen, welche aus sulfonierten Polymerblends (Mischungen) hergestellt wurden. Wie Fig. 3 zeigt, führt die Einführung von sulfonierten Gruppen in ein Polysulfon auch zu einer Abnahme des Kontaktwinkels und damit zu einer besseren Benetzbarkeit der Membranen.

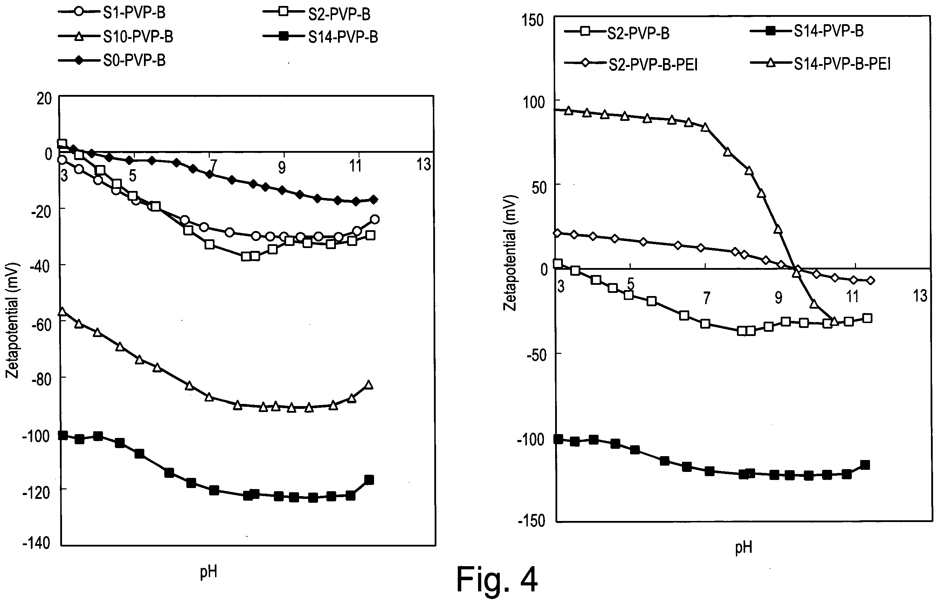

Die erfindungsgemäßen Membranen wurden ferner auch durch eine Analyse der Oberflächenladung (insbesondere Set 1) mittels Zetapotentialmessungen charakterisiert, wie in Fig. 4 dargestellt ist. Fig. 4 zeigt, dass eine Membran aus einem Polymer, das einen höheren Sulfonierungsgrad aufweist, auch einen Anstieg des absoluten Zetapotentialwertes im Vergleich zu Referenzmembranen ohne sulfoniertes Polymer oder mit einem Anteil eines Polymers mit geringerem Sulfonierungsgrad zur Folge hat. Ein höherer Sulfonierungsgrad induziert eine Erhöhung der negativen Nettoladung der Oberfläche. Somit führt die Gegenwart einer höheren Ladungsdichte auf der Membranoberfläche verständlicherweise auch zu einer größeren Polyelektrolytmengenabscheidung (zumindest für PEI, wie in Fig. 4 gezeigt). Alternativ könnte eine höhere - pro Fläche mit einer größeren Sulfonsäuregruppendichte - adsorbierte PEI Menge auch für einen höheren Anteil an ungebundenen Aminogruppen (mehr Loops) sorgen.

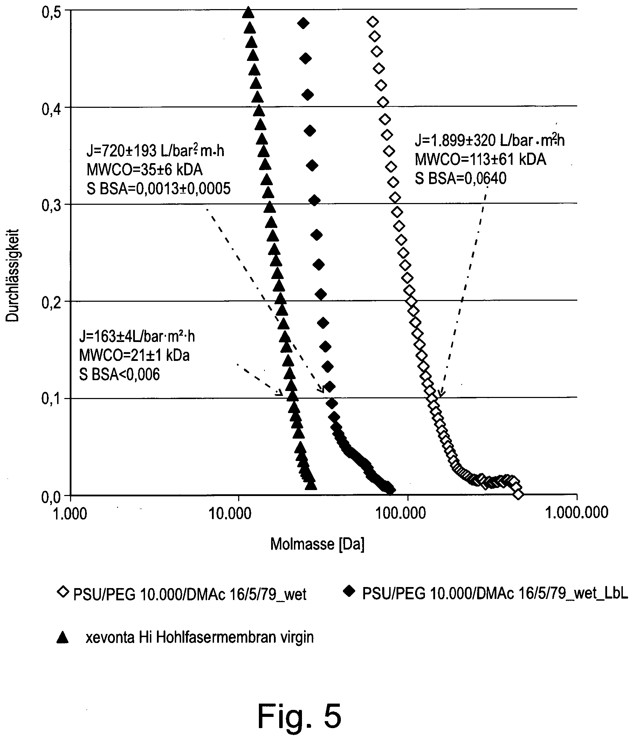

Fig. 5 zeigt ergänzend die Dextran-Siebkurve vor und nach LbL-Modifizierung der Membran P25 aus Set 4. In Folge der LbL-Modifizierung verschiebt sich die Siebkurve deutlich in einen kleineren Molmassen-Bereich. Der Kurvenverlauf wird scheinbar steiler, hin zu einer scharfen Trenngrenze. Daraus ist anzunehmen, dass die Porengrößen nach Modifizierung enger verteilt vorliegen. Mit einem MWCO von 35±6 kDa zeigt diese im Vergleich zu einer "state of the art" xevonta Hi Membran eine bessere Performance für die Anwendung in der Dialyse, auch im Hinblick auf die Wasser-Permeabilität (720 ± 193 L*bar*h-1*m-2 vs. 163 ± 4 L*bar*h-1*m-2) und den Siebkoeffizienten mit BSA @ 22 ± 2°C (0,0013 ± 0,0005 vs. <0,006). Die Basismembran hingegen ist für eine Anwendung in der Dialyse nicht geeignet (vgl. Fig. 5).

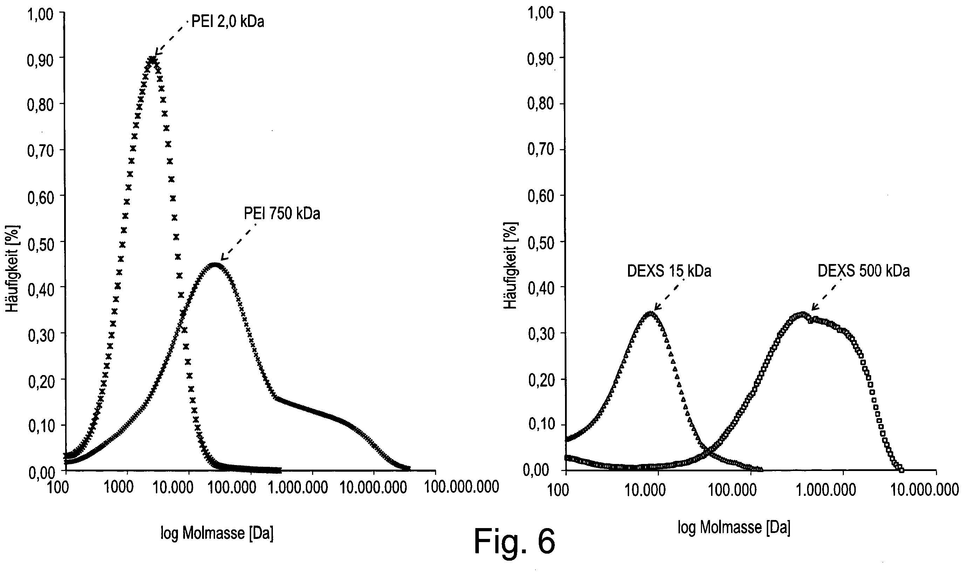

Ergänzend ist in Fig. 6 die Molmassenverteilung für PEI mit 2,0 kDa und 750 kDa sowie DEXS mit 15 kDa und 500 kDa, welche für die LbL-Modifizierung verwendet werden, dargestellt. Für PEI mit 2,0 kDa liegt die Molmasse relativ eng verteilt vor. Im Gegensatz dazu ist für PEI mit 750 kDa die Molmasse sehr breit verteilt mit Fraktionen über nahezu den gesamten Bereich, so dass sich die Verteilungskurven des PEI 750 kDa mit der von 2,0 kDa überschneiden. Das bedeutet, es sind große Anteile kleiner Fraktionen enthalten. Für DEXS ist die Verteilung für 15 kDa relativ eng. Für 500 kDa ergibt sich ein ähnliches Bild, die Molmassen sind ebenfalls relativ eng verteilt. Insgesamt ist die Überschneidung jedoch verglichen mit PEI gering.

Desweiteren zeigen die Ergebnisse, dass die Wasser-Permeabilität, der MWCO und der Siebkoeffizient für BSA der Kompositmembranen durch die LbL-Beschichtung gezielt eingestellt werden kann. Die erfindungsgemäß durch LbL-Beschichtung erhaltenen Membranen sind somit für die Reinigung von Blut bzw. Dialyse geeignet.

Für die Zwecke der vorliegenden Erfindung sei noch bemerkt, dass die Molekularmassenbestimmungen durch Gelpermeationschromatographie [GPC] kalibriert wurden mittels PMMA-Standards für den Molmassenbereich von 0, 1 bis 1200 kDa. Mit den eingesetzten GPC-Säulen wird eine näherungsweisen lineare Kalibrierfunktion im Molmassenbereich von 0,5 bis 1000 kDa erhalten und ist auch sehr gut für die Analyse von Oligomeren geeignet.

**Patentansprüche**

1. Dialysemembran in Hohlfasermembran- oder Flachmembran-Geometrie, aus einem Komposit, welcher aus wenigstens einer Basismembran auf Basis wenigstens eines Polysulfons mit wenigstens einem porenbildenden hydrophilen Additiv und wenigstens einer auf der Basismembran angeordneten Funktionalschicht aufgebaut ist, wobei die Funktionalschicht aus wenigstens einer Schicht aus einem polymeren polykationischen Haftvermittler und wenigstens einer weiteren Schicht aus einem polymeren Polyanion gebildet ist,
**dadurch gekennzeichnet, dass**
die Basismembran aus einem Material gebildet ist, welches ausgewählt ist aus: einem Polyamid [PA], einem Polyacrylnitril [PAN], einem Polymethylmethacrylat [PMMA], einer Polyacrylsäure [PAA], einem Polycarbonat [PC], einem Polyurethan [PUR], einem Polysulfon [PSU], einem sulfonierten Polysulfon [SPSU], einem Polyethersulfon [PES], einem sulfonierten Polyethersulfon [SPES], einem Polyphenylsulfon [PPSU], einem sulfonierten Polyphenylsulfon [SPPSU]; sowie Mischungen daraus;
der polykationische Haftvermittler ausgewählt ist aus der Gruppe, bestehend aus: Polyethylenimin [PEI], Chitosan, Polylysin, Polyarginin, Polyornithin oder einer Mischung daraus;
das Polyanion ein carboxyliertes Polysaccharid ist oder sulfatiertes Polysaccharid ist, welches ausgewählt ist aus: einem Dextransulfat mit einer Molekularmasse ($M_w$) von 15 kDa bis 1 MDa, einem sulfatierten Chitosan mit einer Molekularmasse ($M_w$) von 30 kDa bis 750 kDa; einem Cellulosesulfat mit einer Molekularmasse ($M_w$) zwischen 20 kDa und 1 MDa, vorzugsweise ca. 100 kDa; oder Mischungen daraus; und
wobei das porenbildende hydrophile Additiv ausgewählt ist aus:

Polyvinylpyrrolidon [PVP], einem kurzkettigen Glykol mit 2 bis 10 C-Atomen, Triethylenglykol, Propylenglykol, Polyethylenglykol [PEG]/Polyethylenoxid [PEO] sowie Mischungen daraus.

2. Dialysemembran nach Anspruch 1, **dadurch gekennzeichnet, dass** das SPSU einen Sulfonierungsgrad von 0,1 bis 20 Gew.-%,bezogen auf das Gewicht des unsulfonierten PSUs aufweist;

und/oder

das SPES einen Sulfonierungsgrad von 0,1 bis 20 Gew.-%, bezogen auf das Gewicht des unsulfonierten PES aufweist;

und/oder

das SPPSU einen Sulfonierungsgrad von 0,1 bis 20 Gew.-%, bezogen auf das Gewicht des unsulfonierten PPSUs aufweist.

3.  Dialysemembran nach Anspruch 2, **dadurch gekennzeichnet, dass** das SPSU einen Sulfonierungsgrad von 9,3 oder 13,4 Gew.-%, bezogen auf das Gewicht des unsulfonierten PSUs aufweist.

4.  Dialysemembran nach Anspruch 2, **dadurch gekennzeichnet, dass** das SPES einen Sulfonierungsgrad von 1,1; 3,6 oder 14,1 Gew.-%, bezogen auf das Gewicht des unsulfonierten PES aufweist.

5.  Dialysemembran nach Anspruch 2, **dadurch gekennzeichnet, dass** das SPPSU einen Sulfonierungsgrad von 1,0, 2,0, 10,1 oder 14,7 Gew.-%, bezogen auf das Gewicht des unsulfonierten PPSUs aufweist.

6.  Dialysemembran aus einem Komposit nach einem der vorhergehenden Ansprüche, **dadurch gekennzeichnet, dass** sie eine hydraulische Permeabilität von 10 bis 2.000 L/bar*h*m$^2$ und einen Siebkoeffizienten (@22±2°) für BSA im Bereich von 0,0001 bis 0,5 sowie einen Molekularmasse-Ausschlusswert von 20 bis 50 kDa aufweist.

7.  Dialysemembran in Flach- oder Hohlfasermembrangeometrie nach einem der vorhergehenden Ansprüche, **dadurch gekennzeichnet, dass** sie eine Wandstärke von 20 bis 200 μm aufweist.

8.  Dialysemembran in Hohlfasermembrangeometrie nach einem der vorhergehenden Ansprüche, **dadurch gekennzeichnet, dass** sie einen Lumendurchmesser von 100 bis 400 μm aufweist.

9.  Verfahren zur Herstellung einer Dialysemembran in Hohlfasermembran- oder Flachmembran-Geometrie gemäß wenigstens einem der Ansprüche 1 bis 8, wobei

     a) man zur Herstellung einer Basismembran für die Dialysemembran eine Gieß- oder Spinnlösung aus wenigstens einem Polysulfon, welches ausgewählt ist aus: einem Polysulfon [PSU], einem sulfonierten Polysulfon [SPSU], einem Polyethersulfon [PES], einem sulfonierten Polyethersulfon [SPES], einem Polyphenylsulfon [PPSU], einem sulfonierten Polyphenylsulfon [SPPSU]; sowie Mischungen daraus; und wenigstens einem porenbildenden hydrophilen Additiv in wenigstens einem organischen Lösungsmittel, ausgewählt aus N,N-Dimethylacetamid, N-Methyl-2-pyrrolidon herstellt; und

     b) man die so hergestellte Polymermischungs-Lösung zur Ausbildung der Basismembran mit einem Fällungsagens in Kontakt bringt, nach der Präzipitation der Polymermischung in Flach- oder Hohlfaserform das organische Lösungsmittel auswäscht; und

     c) man die in Schritt b) hergestellte Basismembran zur Erzeugung einer Funktionalschicht darauf einer Oberflächenmodifikation unterzieht, indem man wenigstens eine Schicht-um-Schicht- [layer-by-layer] Abscheidung auf der Oberfläche der Basismembran durchführt, um die Dialysemembran zu erhalten, wobei man als erste Schicht auf der Oberfläche der Basismembran wenigstens einen polymeren polykationischen Haftvermittler aufbringt und auf der polykationischen Schicht als zweite Schicht wenigstens ein polymeres Polyanion aufbringt; und wobei

     d) der polykationische Haftvermittler ausgewählt wird aus der Gruppe, bestehend aus Polyethylenimin [PEI], Chitosan, Polylysin, Polyarginin und Polyornithin, sowie Mischungen daraus ; und

     als Polyanion ein carboxyliertes Polysaccharid oder ein sulfatiertes Polysaccharid eingesetzt wird, welches ausgewählt wird, aus der Gruppe, bestehend aus: einem Dextransulfat mit einer Molekularmasse (M$_w$) von 15 kDa bis 1 MDa, einem sulfatierten Chitosan mit einer Molekularmasse (M$_w$) von 30 kDa bis 750 kDa; einem Cellulosesulfat mit einer Molekularmasse (M$_w$) zwischen 20 kDa und 1 MDa, vorzugsweise ca. 100 kDa; oder Mischungen daraus; und

     wobei das porenbildende hydrophile Additiv ausgewählt ist aus:

     Polyvinylpyrrolidon [PVP], einem kurzkettigen Glykol mit 2 bis 10 C-Atomen, Triethylenglykol, Propylenglykol, Polyethylenglykol [PEG]/Polyethylenoxid [PEO] sowie Mischungen daraus.

10. Verfahren nach Anspruch 9, **dadurch gekennzeichnet, dass** die Nettoladung der Oberfläche der Basismembran sowohl durch Anteile von sulfoniertem Polymer, insbesondere deren Sulfonierungsgrad, sowie den aufgebrachten Polyelektrolyten (polymerer polykationischer Haftvermittler und polymeres Polyanion), insbesondere deren Polyelektrolytmengen, eingestellt werden kann.

11. Verfahren nach Anspruch 9 oder 10, **dadurch gekennzeichnet, dass** das Polyethylenglykol/Polyethylenoxid eine Molekularmasse von 600 Da bis 500 kDa ($M_w$), insbesondere ca. 10 KDa ($M_w$) oder 8 kDa ($M_n$), aufweist und/oder das PVP eine Molekularmasse ($M_w$) 1kDa bis 2,2 MDa, insbesondere ca. 1,1 MDa, aufweist.

12. Verfahren nach einem der Ansprüche 9 bis 11, **dadurch gekennzeichnet, dass** der Haftvermittler ein PEI mit einer Molekularmasse ($M_w$) von 1 kDa bis 2 MDa, insbesondere ca. 2 kDa, bevorzugt ca. 25 kDa, vorzugsweise ca. 750 kDa oder ein C2 bis C8-Dialkanal-vernetztes, insbesondere 1,5-Pentandial-vernetztes, hochmolekulares PEI ist, vorzugsweise ein solches ist, welches aus einem PEI mit einer Molekularmasse von ca. 2,0 kDa ($M_n$) durch Vernetzung mit 1,5-Pentandial herstellbar ist.

13. Verfahren nach einem der Ansprüche 9 bis 12, **dadurch gekennzeichnet, dass** das Polysaccharid ein Dextransulfat mit einer Molekularmasse ($M_w$) von ca. 500 kDa und/oder ein sulfoniertes Chitosan mit einer Molekularmasse ($M_w$) von 30 kDa bis 750 KDa, ist.

14. Dialysemembran, erhältlich nach einem Verfahren gemäß wenigstens einem der Ansprüche 9 bis 13.

15. Dialysemembran-Modul, **dadurch gekennzeichnet, dass** es als Füllung Dialysemembranen gemäß wenigstens einem der Ansprüche 1 bis 8 enthält.

Fig. 1

Fig. 2

Fig. 3

Fig. 4

Fig. 5

Fig. 6

Europäisches
Patentamt

European
Patent Office

Office européen
des brevets

# EUROPÄISCHER RECHERCHENBERICHT

**Nummer der Anmeldung**

EP 17 15 6674

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | FILIZ YASAR MAHLICLI ET AL: "Surface modification of polysulfone based hemodialysis membranes with layer by layer self assembly of polyethyleneimine/alginate-heparin: a simple polyelectrolyte blend approach for heparin immobilization", JOURNAL OF MATERIALS SCIENCE: MATERIALS IN MEDICINE, Bd. 24, Nr. 2, 1. Februar 2013 (2013-02-01), Seiten 533-546, XP055381655, United States ISSN: 0957-4530, DOI: 10.1007/s10856-012-4804-2 * das ganze Dokument * ----- | 1-15 | INV. B01D61/28 B01D67/00 B01D69/02 B01D71/68 B01D69/12 |
| X,D | KOCHAN J ET AL: "Properties of polyethersulfone ultrafiltration membranes modified by polyelectrolytes", DESALINATION, ELSEVIER, AMSTERDAM, NL, Bd. 250, Nr. 3, 30. Januar 2010 (2010-01-30), Seiten 1008-1010, XP026788767, ISSN: 0011-9164, DOI: 10.1016/J.DESAL.2009.09.092 [gefunden am 2009-10-22] * das ganze Dokument * ----- -/-- | 1-15 | |

**RECHERCHIERTE SACHGEBIETE (IPC)**

B01D

Der vorliegende Recherchenbericht wurde für alle Patentansprüche erstellt

| Recherchenort | Abschlußdatum der Recherche | Prüfer |
|---|---|---|
| München | 10. August 2017 | Kukolka, Florian |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes
    Dokument

EPO FORM 1503 03.82 (P04C03)

Seite 1 von 2

Europäisches
Patentamt

European
Patent Office

Office européen
des brevets

# EUROPÄISCHER RECHERCHENBERICHT

Nummer der Anmeldung

EP 17 15 6674

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | ARAVIND ET AL: "Transport studies of BSA, lysozyme and ovalbumin through chitosan/polystyrene sulfonate multilayer membrane", JOURNAL OF MEMBRANE SCIENCE, ELSEVIER BV, NL, Bd. 299, Nr. 1-2, 22. Juni 2007 (2007-06-22), Seiten 146-155, XP022130739, ISSN: 0376-7388, DOI: 10.1016/J.MEMSCI.2007.04.036 * das ganze Dokument * ----- | 1-15 | |
| A | WO 03/020425 A1 (POREX CORP [US]) 13. März 2003 (2003-03-13) * Anspruch 5 * ----- | 1-15 | |
| A | KRASEMANN L ET AL: "Self-assembled polyelectrolyte multilayer membranes with highly improved pervaporation separation of ethanol/water mixtures", JOURNAL OF MEMBRANE SCI, ELSEVIER BV, NL, Bd. 181, Nr. 2, 30. Januar 2001 (2001-01-30), Seiten 221-228, XP004228113, ISSN: 0376-7388, DOI: 10.1016/S0376-7388(00)00535-4 * das ganze Dokument * ----- | 1-15 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 10. August 2017 | Kukolka, Florian |

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**

EP 17 15 6674

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-08-2017

| Im Recherchenbericht angeführtes Patentdokument | Datum der Veröffentlichung | Mitglied(er) der Patentfamilie | | Datum der Veröffentlichung |
|---|---|---|---|---|
| WO 03020425 A1 | 13-03-2003 | AT | 353707 T | 15-03-2007 |
| | | CN | 1578702 A | 09-02-2005 |
| | | DE | 60218187 T2 | 31-10-2007 |
| | | EP | 1427532 A1 | 16-06-2004 |
| | | JP | 4652684 B2 | 16-03-2011 |
| | | JP | 2005501758 A | 20-01-2005 |
| | | US | 2003096424 A1 | 22-05-2003 |
| | | US | 2003124332 A1 | 03-07-2003 |
| | | US | 2006280931 A1 | 14-12-2006 |
| | | WO | 03020425 A1 | 13-03-2003 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20130277878 A1
- DE 102004008220 B4
- US 6042783 A
- EP 1634611 B1
- CN 201669064 U
- WO 2013156598 A1
- WO 2013156597 A1

**In der Beschreibung aufgeführte Nicht-Patentliteratur**

- **STEFAN HEINRICH ; H. OLIVER PFIRRMANN.** *Nanotechnologie für die Gesundheit, Gesundheitsvorsorge im Wandel,* 2010, vol. 2, 12
- **BLANCO et al.** *J Appl Polymer Sci,* 2002, vol. 84, 2461
- **LI et al.** *J Membrane Sci,* 2008, vol. 309, 45
- **MAHLICLI et al.** *J Mater Sci: Med,* 2013, vol. 24, 533
- **MALAISAMY et al.** *Langmuir,* 2005, vol. 21, 10587
- **KOPEC et al.** *J Membrane Sci,* 2011, vol. 369, 59
- **KOCHAN et al.** *Desalination,* 2010, vol. 250, 1008
- **WANG et al.** *Macromol. Symp.,* 2001, vol. 175, 387-395
- **JEFFREY B. MECHAM.** Dissertation. Fakultät für Chemie des Virginia Polytechnic Institute and State University, 23. April 2001
- **SAECHTLING.** Kunststofftaschenbuch. Carl Hanser Verlag, 2013